(19) Europäisches Patentamt
European Patent Office
Office européen des brevets

(11) Numéro de publication : **0 303 826 B1**

# (12) FASCICULE DE BREVET EUROPEEN

(45) Date de publication du fascicule du brevet : **27.02.91 Bulletin 91/09**

(51) Int. Cl.⁵ : **C07C 211/52,** C07C 211/53, C07C 215/28, C07C 215/80, C07C 217/84, C07C 323/09, C09B 51/00, A61K 7/13

(21) Numéro de dépôt : **88111198.3**

(22) Date de dépôt : **13.07.88**

## (54) 2-Nitro métaphénylènediamines substituées, leur procédé de préparation et leur utilisation en teinture des fibres kératiniques et en particulier des cheveux humains.

(30) Priorité : **27.07.87 LU 86951**

(43) Date de publication de la demande : **22.02.89 Bulletin 89/08**

(45) Mention de la délivrance du brevet : **27.02.91 Bulletin 91/09**

(84) Etats contractants désignés : **AT BE CH DE ES FR GB GR IT LI NL SE**

(56) Documents cités :
**EP-A- 0 203 446**
**GB-A- 2 090 853**
**GB-A- 2 186 586**
**GB-A- 2 186 587**

(73) Titulaire : **L'OREAL**
**14, Rue Royale**
**F-75008 Paris (FR)**

(72) Inventeur : **Junino, Alex**
**16, Rue Docteur Bergonié**
**F-93180 Livry-Gargan (FR)**
Inventeur : **Jehanno, Nicole**
**49 Rue des Lièvres**
**F-91800 Brunoy (FR)**
Inventeur : **Lang, Gérard**
**44, Avenue Lacor**
**F-95210 Saint-Gratien (FR)**

(74) Mandataire : **Casalonga, Axel et al**
**BUREAU D.A. CASALONGA - JOSSE**
**Morassistrasse 8**
**D-8000 München 5 (DE)**

**EP 0 303 826 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention est relative à de nouvelles 2-nitro métaphénylènediamines substituées, à leur procédé de préparation, aux compositions tinctoriales pour fibres kératiniques et en particulier pour cheveux humains les contenant et à un procédé de teinture utilisant lesdites compositions tinctoriales.

Il est bien connu que, pour conférer aux cheveux une coloration directe ou des reflets complémentaires dans le cas de la coloration d'oxydation, on peut utiliser les dérivés nitrés de la série benzénique.

La demande GB 2 090 853 (L'OREAL) décrit des 3-nitro orthophénylènediamines portant éventuellement un substituant alkyle en méta du groupe nitro, conférant aux cheveux des couleurs jaune-orangé.

La demande de brevet EP 0 203 446 décrit quant à elle des nitroparaphénylènediamines portant en position para du groupement nitro, un substituant alcoxy ou hydroxyalcoxy. Ces nitroparaphénylènediamines conduisent à des couleurs allant du rouge au violet.

On a déjà préconisé l'utilisation en teinture directe, selon les brevets français 1 508 405 et 1 584 965 de la demanderesse, de 4-nitro métaphénylènediamines éventuellement substituées en position 6 par des radicaux alkyle ou alcoxy inférieurs ou par un atome d'halogène.

Au cours de ses recherches, la demanderesse a découvert qu'il était possible d'obtenir des teintures capillaires présentant une très bonne stabilité à la lumière, aux lavages et aux intempéries en utilisant une famille particulière de 2-nitro métaphénylènediamines substituées de formule :

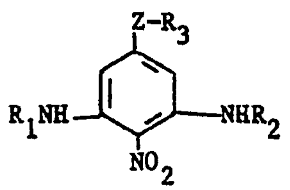

(I)

dans laquelle Z désigne -O-, -S- ou -NH- et

$R_1$, $R_2$, et $R_3$, identiques ou différents, désignent un atome d'hydrogène, un radical alkyle, hydroxyalkyle, polyhydroxyalkyle, alcoxyalkyle, hydroxyalcoxyalkyle, aminoalkyle dont le radical amino peut être mono-ou disubstitué par un radical alkyle ou hydroxyalkyle.

Les radicaux alkyle et alcoxy mentionnés ci-dessus comportent 1 à 6 atomes de carbone.

La présente invention a donc pour objet les nouveaux composés de formule (I) ainsi que leurs sels cosmétiquement acceptables.

Parmi les groupements $R_1$, $R_2$ et $R_3$ particulièrement préférés, on peut citer l'hydrogène, les radicaux méthyle, éthyle, n-propyle, n-butyle, β-hydroxyéthyle, β-hydroxypropyle, γ-hydroxypropyle, β, γ-dihydroxypropyle, méthoxyéthyle, éthoxyéthyle, β-hydroxyéthoxyéthyle, β-aminoéthyle, β-hydroxyéthylaminoéthyle, β-diéthylaminoéthyle.

A titre de composés de formule (I) particulièrement préférés selon l'invention, on peut citer : le 2,6-diamino-4-méthoxynitrobenzène, le 2,6-diamino-4-hydroxynitrobenzène, le 2-(β-hydroxyéthyl) amino-6-(β-hydroxyéthyl) amino-4-méthoxynitrobenzène, le 2-[(3-méthylamino-5-méthylamino-4-nitro) phénoxy] éthanol, le 3-[(3-méthylamino-5- méthylamino-4-nitro) phénoxy] propane 1,2-diol, le 2-méthylamino-6-(β-méthoxyéthyl) amino-4-méthoxynitrobenzène, le 2-méthylamino-6-méthylamino-4-(β-hydroxypropyl) aminonitrobenzène, le 2-méthylamino-6-méthylamino-4-(β, γ -dihydroxypropyl) aminonitrobenzène, le 2-méthylamino-6-méthylamino-4-[(β-hydroxyéthoxy) éthylamino] nitrobenzène, le 2-(β-hydroxyéthyl) amino-6-(β-hydroxyéthyl)amino-4-(β-aminoéthyl) aminonitrobenzène, le 2-(β-hydroxyéthyl)amino-4-(β-hydroxyéthyl) amino-6-(β-hydroxyéthyl) aminonitrobenzène, le 4-β-méthoxyéthoxy-2-(β-hydroxyéthyl) amino-6-(β-hydroxyéthyl)aminonitrobenzène, le [3-(β-hydroxyéthyl) amino-5- (β-hydroxyéthyl)amino-4-nitrophényl] β-hydroxyéthylthioéther, le 2-(β, γ -dihydroxypropyl) amino-6-(β, γ-dihydroxypropyl) amino-4-méthoxynitrobenzène, le 2-(γ-hydroxypropyl) amino-4-(γ-hydroxypropyl) amino-6-(γ-hydroxypropyl) aminonitrobenzène, le 2-amino-6-(β-hydroxyéthyl) amino-4-méthoxynitrobenzène ainsi que les sels cosmétiquement acceptables de ces composés.

Un autre objet de l'invention est constitué par le procédé de préparation des composés de formule (I) qui consiste à disubstituer un 2,4,6-trihalogénonitrobenzène pour obtenir un 2,6-diamino-4-halogénonitrobenzène qu'on fait ensuite réagir, avec HZ $R_3$ pour obtenir le composé de formule (I).

Ce procédé peut être schématisé de la façon suivante :

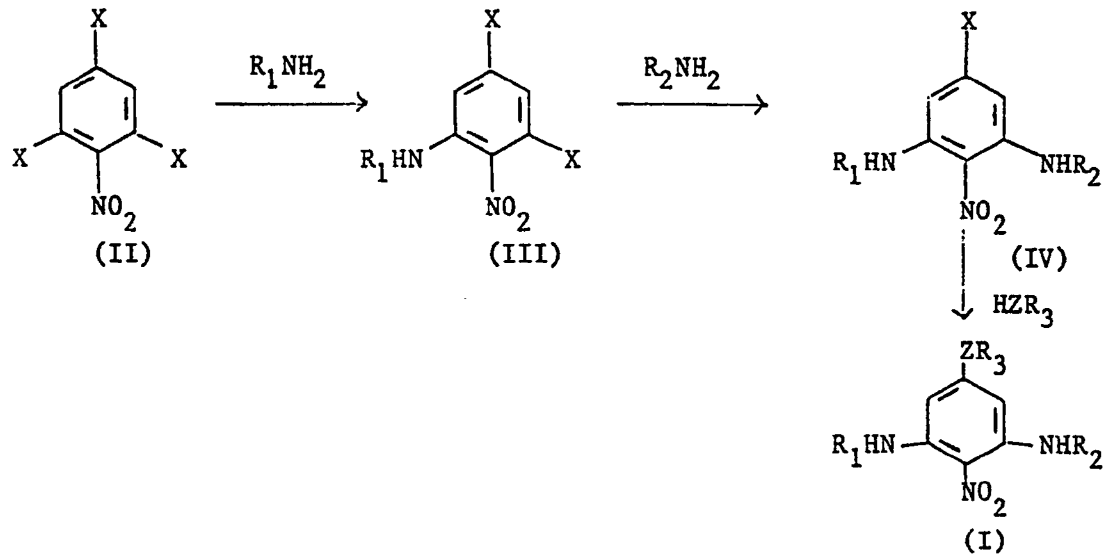


(II) (III) (IV) (I)

X représentant un atome d'halogène, et en particulier le chlore ou le fluor, $R_1$, $R_2$, $R_3$ et Z ayant les significations indiquées ci-dessus.

Les composés de formule (IV) peuvent être préparés en faisant réagir dans un premier temps l'ammoniac ou une amine de formule $NH_2R_1$, $R_1$ ayant les significations ci-dessus définies, sur le 2,4,6-trihalogénonitrobenzène de formule (II) pour obtenir le composé (III) qui lui-même soumis à l'action d'une amine $NH_2R_2$ ou d'ammoniac conduit dans un deuxième temps au composé (IV).

Le composé (IV) pour lequel le groupement $R_2$ est identique au groupement $R_1$ est préparé en une seule étape à partir du composé (II). La synthèse du 2,6-diamino-4-chloronitrobenzène par action d'ammoniac sur le 2,4-6-trichloronitrobenzène est connue (Beil., vol. 13, p. 58).

La substitution des groupements halogéno par les groupements amino $NHR_1$ et $NHR_2$ est effectuée en présence ou non de solvants. Les solvants couramment utilisés sont les alcools inférieurs ou les solvants tels que le diméthylformamide, le diméthylsulfoxyde, la N-méthylpyrrolidone ou la N, N'-diméthylpropylène urée. Dans le cas où l'ammoniac ou l'amine $NH_2R_1$ ou $NH_2R_2$ sont utilisés en solution aqueuse, il est préférable, pour des raisons de solubilité, d'ajouter un tiers solvant choisi parmi ceux cités ci-dessus.

Les températures auxquelles sont effectuées les réactions de substitution des groupements halogéno par les groupements amino-$NHR_1$ et/ou -$NHR_2$ peuvent varier entre –10°C et la température de reflux de l'amine $NH_2R_1$ et/ou $NH_2R_2$ ou de celle du solvant. Généralement, la température est comprise entre 20°C et 170°C.

Dans le cas d'ammoniac gazeux ou d'amines $NH_2R_1$ et/ou $NH_2R_2$ à point d'ébullition bas, la substitution peut être effectuée dans un autoclave, une pression allant jusqu'à 25 kg/cm$^2$ étant généralement suffisante. La substitution de l'atome d'halogène en position 4 par le substituant -$ZR_3$ s'effectue en présence ou non de solvants. Dans le cas où Z désigne -NH-, on utilise généralement un excès d'amine $H_2NR_3$ qui joue le rôle de réactif et de solvant.

Dans le cas où Z est un atome d'oxygène ou de soufre, la substitution s'effectue en présence d'un hydroxyde ou d'un alcoolate alcalins, soit en présence d'un solvant comme le dioxane, la N-méthyl pyrrolidone, les alcools inférieurs (en $C_1$-$C_4$) soit en l'absence de solvant, le réactif $HZR_3$ utilisé en excès jouant le rôle de solvant.

La température de réaction est comprise entre 30 et 150°C.

Lorsque -$ZR_3$ représente le radical -OH-, les composés de formule :

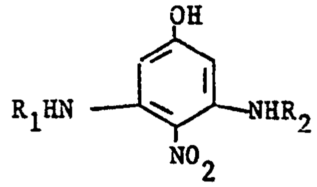

(Ia)

dans laquelle $R_1$ et $R_2$ désignent H ou alkyle, sont obtenus à partir des composés de formule (I) dans laquelle Z est un atome d'oxygène et $R_3$ désigne le radical méthyle, par désalkylation avec l'acide bromhydrique à

une température comprise entre 50 et 100°C.

La présente invention a également pour objet la composition tinctoriale pour fibres kératiniques et en particulier pour cheveux humains, contenant dans un milieu solvant approprié pour procéder à la teinture des fibres kératiniques, au moins un colorant direct de formule (I), ces compositions tinctoriales étant mises en oeuvre dans un procédé de coloration directe ou dans un procédé de teinture d'oxydation.

Un autre objet de l'invention est donc constitué par le procédé de coloration des fibres kératiniques et plus particulièrement des cheveux humains, utilisant la composition tinctoriale contenant le colorant de formule (I).

Les compositions tinctoriales conformes à l'invention contiennent, dans un milieu solvant, au moins un composé répondant à la formule (I) ou l'un de ses sels cosmétiquement acceptables, et peuvent être utilisées pour la coloration directe des fibres kératiniques ou pour la coloration d'oxydation de ces fibres, auquel cas les composés de formule (I) confèrent des reflets complémentaires à la coloration de base obtenue par développement oxydant de précurseurs de colorants d'oxydation.

Les compositions tinctoriales selon l'invention contiennent les composés (I) dans des proportions comprises entre 0,001 et 5% en poids et de préférence entre 0,05 et 2% en poids par rapport au poids total de la composition.

Le milieu solvant est de préférence un véhicule cosmétique généralement constitué par de l'eau, mais on peut également ajouter, dans les compositions, des solvants organiques pour solubiliser des composés qui ne seraient pas suffisamment solubles dans l'eau. Parmi ces solvants, on peut citer les alcanols inférieurs tels que l'éthanol et l'isopropanol, les alcools aromatiques comme l'alcool benzylique et le phénoxyéthanol, les polyols tels que le glycérol, les glycols et leurs éthers comme le 2-butoxy éthanol ou le 2-éthoxy éthanol, l'éthylèneglycol, le propylèneglycol, le monométhyléther et le monoéthyléther du diéthylèneglycol ainsi que les produits analogues et leurs mélanges. Ces solvants sont de préférence présents dans des proportions allant de 1 à 75% en poids et en particulier de 5 à 50% en poids par rapport au poids total de la composition.

Ces compositions peuvent contenir des agents tensio-actifs anioniques, cationiques, non-ioniques, amphotères ou leurs mélanges. Ces produits tensio-actifs sont présents dans les compositions de l'invention dans des proportions comprises entre 0,5 et 55% en poids et de préférence entre 4 et 40% en poids par rapport au poids total de la composition.

Les compositions peuvent être épaissies de préférence avec des composés choisis parmi l'alginate de sodium, la gomme arabique, la gomme de xanthane, les dérivés de la cellulose tels que la méthylcellulose, l'hydroxyéthylcellulose, l'hydroxypropylméthylcellulose, la carboxyméthylcellulose et les polymères divers ayant la fonction d'épaississant tels que plus particulièrement les dérivés d'acide acrylique. Il est également possible d'utiliser des agents épaississants minéraux tels que la bentonite. Ces agents épaississants sont présents de préférence dans des proportions comprises entre 0,1 et 10% en poids et en particulier entre 0,5 et 2% en poids par rapport au poids total de la composition.

Les compositions selon l'invention peuvent également contenir divers adjuvants habituellement utilisés dans les compositions tinctoriales pour cheveux et en particulier des agents de pénétration, des agents dispersants, des agents séquestrants, des agents filmogènes, des tampons et des parfums.

Ces compositions peuvent se présenter sous des formes diverses telles que liquide, crème, gel ou toute autre forme appropriée pour réaliser une teinture des cheveux. Elles peuvent en outre être conditionnées en flacons aérosols en présence d'un agent propulseur.

Le pH de ces compositions tinctoriales peut être compris entre 3 et 11, 5, de préférence entre 5 et 11, 5. On l'ajuste à la valeur souhaitée à l'aide d'un agent alcalinisant tel que l'ammoniaque, le carbonate de sodium, de potassium ou d'ammonium, les hydroxydes de sodium ou de potassium, les alcanolamines telles que la mono-, la di-ou la triéthanolamine, le 2-amino-2-méthyl propan-1-ol, le 2-amino-2-méthyl propane-1,3-diol, les alkylamines telles que l'éthylamine ou la triéthylamine ou à l'aide d'un agent d'acidification tel que les acides phosphorique, chlorhydrique, tartrique, acétique, lactique ou citrique.

Lorsque les compositions sont destinées à être utilisées dans un procédé de coloration directe des cheveux, elles peuvent contenir en plus des composés conformes à l'invention, d'autres colorants directs tels que des colorants azoïques ou anthraquinoniques, comme par exemple la 1,4,5,8-tétra-amino anthraquinone, des indophénols, des indoanilines et des colorants nitrés de la série benzénique différents des composés de formule (I).

Les concentrations de ces colorants directs autres que les colorants de formule (I) peuvent être comprises entre 0,001 et 5% en poids par rapport au poids total de la composition.

Ces compositions, mises en oeuvre dans un procédé de teinture par coloration directe, sont appliquées sur les fibres kératiniques pendant un temps de pose variant de 5 à 50 minutes, puis les fibres sont rincées, éventuellement lavées au shampooing, rincées à nouveau et séchées.

Les compositions selon l'invention peuvent également être mises en oeuvre sous forme de lotions capillaires de mise en plis destinées tout à la fois à conférer aux cheveux une légère coloration ou des reflets et à améliorer la tenue de la mise en plis. Dans ce cas, elles se présentent sous forme de solutions aqueuses, alcooliques ou hydroalcooliques renfermant au moins une résine cosmétique et leur application s'effectue sur des cheveux humides préalablement lavés et rincés qui sont éventuellement enroulés puis séchés.

Les résines cosmétiques utilisées dans les lotions de mise en plis peuvent être en particulier la polyvinylpyrrolidone, les copolymères acide crotonique-acétate de vinyle, vinylpyrrolidone-acétate de vinyle, les semi-esters de l'anhydride maléique-éther butylvinylique ou de l'anhydride maléique-éther méthylvinylique ou les copolymères acide maléïque/ éther méthyl- ou butylvinylique, ainsi que tout autre polymère cationique, anionique, non ionique ou amphotère habituellement utilisé dans ce type de composition. Ces résines cosmétiques entrent dans les compositions de l'invention à raison de 0,1 à 4% en poids, et de préférence de 1 à 3% en poids sur la base du poids total de la composition.

Lorsque les compositions selon l'invention constituent des teintures d'oxydation impliquant la révélation par un oxydant, les composés de formule (I) conformes à l'invention sont essentiellement utilisés en vue d'apporter des reflets à la teinture finale.

Ces compositions contiennent alors en association avec au moins un colorant nitré de formule (I) et éventuellement d'autres colorants directs, des précurseurs de colorants par oxydation.

Elles peuvent contenir par exemple des paraphénylènediamines telles que : la paraphénylènediamine, la paratoluylènediamine, la 2-chloro paraphénylènediamine, la 2,6-diméthyl paraphénylènediamine, la 2,6-diméthyl-3-méthoxy paraphénylènediamine, la N-(β-méthoxyéthyl) paraphénylènediamine, la N, N-(β-hydroxyéthyl) paraphénylènediamine, la N, N-(éthyl, carbamylméthyl)-4-amino aniline, ainsi que leurs sels.

Elles peuvent également contenir des paraaminophénols, par exemple : le paraaminophénol, le N-méthylparaaminophénol, le 2-chloro-4-amino phénol, le 3-chloro-4-amino phénol, le 2-méthyl-4-amino phénol, et leurs sels.

Elles peuvent également contenir de l'orthoaminophénol.

Elles peuvent aussi contenir des dérivés hétérocycliques, tels que par exemple : la 2,5-diamino pyridine, la 7-amino benzomorpholine.

Les compositions selon l'invention peuvent contenir en association avec les précurseurs de colorants par oxydation, des coupleurs bien connus dans l'état de la technique.

A titre de coupleurs, on peut citer notamment : les métadiphénols, les métaaminophénols et leurs sels, les métaphénylènediamines et leurs sels, les métaacylaminophénols, les méta uréidophénols, les méta-carbalcoxyaminophénols.

On peut enfin mentionner comme autres coupleurs utilisables dans les compositions de l'invention : l'α-naphtol, les coupleurs possédant un groupement méthylène actif tels que les composés dicétoniques et les pyrazolones et les couleurs hétérocycliques dérivés de la pyridine et de la benzomorpholine.

Ces compositions contiennent, en plus des précurseurs de colorants par oxydation, des agents réducteurs présents dans des proportions comprises entre 0,05 et 3% en poids par rapport au poids total de la composition.

Les précurseurs de colorants par oxydation peuvent être utilisés, dans les compositions de l'invention, à des concentrations comprises entre 0,001 et 5% en poids et de préférence entre 0,03 et 2% en poids sur la base du poids total de la composition. Les coupleurs peuvent également être présents dans des proportions comprises entre 0,001 et 5% en poids, de préférence entre 0,015 et 2% en poids. Le pH de ces compositions de teinture par oxydation est de préférence compris entre 7 et 11, 5 et est ajusté à l'aide d'agents alcalinisants définis ci-dessus.

Le procédé de teinture des fibres kératiniques, en particulier des cheveux humains, mettant en oeuvre la révélation par un oxydant, consiste à appliquer sur les fibres la composition tinctoriale comprenant à la fois un colorant selon l'invention et les précurseurs de colorants. Le développement de la coloration peut alors s'effectuer lentement en présence de l'oxygène de l'air, mais on utilise de préférence un système révélateur chimique qui est le plus souvent choisi parmi l'eau oxygénée, le peroxyde d'urée et les persels. On utilise en particulier une solution d'eau oxygénée à 20 volumes.

Une fois que l'on a appliqué sur les fibres kératiniques la composition avec l'agent oxydant, on laisse poser pendant 10 à 50 minutes, de préférence 15 à 30 minutes, après quoi on rince les fibres kératiniques, on les lave éventuellement au shampooing, on les rince à nouveau et on sèche.

Les exemples qui suivent sont destinés à illustrer l'invention sans présenter un caractère limitatif.

Exemple de préparation 1

Préparation du 2,6-diamino-4-méthoxynitrobenzène

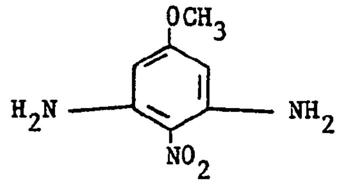

1ère étape :

Préparation du 2,6-diamino-4-chloronitrobenzène

Dans un autoclave, on ajoute 0,34 mole (76 g) de 2,4,6-trichloronitrobenzène à 400 ml d'ammoniac à 28% dans l'eau et 100 ml d'éthanol. Le milieu réactionnel est chauffé 16 heures à 155°-160°C, la pression étant de 20 kg/cm². Après refroidissement le produit attendu précipite. Après essorage et réempâtage dans l'eau jusqu'à neutralité des eaux de lavage, il est séché sous vide en présence de pentaoxyde de phosphore. Après recristallisation de l'isopropanol, afin d'éliminer une résine, il fond à 202°C (littérature 192°-194°C).

L'analyse du produit obtenu donne les résultats suivants :

| Analyse | Calculé pour $C_6H_6ClN_3O_2$ | Trouvé |
|---|---|---|
| C% | 38,40 | 38,55 |
| H% | 3,20 | 3,26 |
| N% | 22,40 | 22,43 |
| O% | 17,06 | 16,88 |
| Cl% | 18,93 | 18,74 |

2ème étape :

Préparation du 2,6-diamino-4-méthoxynitrobenzène

On chauffe 4 heures 30 minutes au reflux, 0,024 mole (4,5 g) de 2,6-diamino-4-chloronitrobenzène dans 9 ml de N-méthylpyrrolidone et 22,5 ml d'une solution à 30% de méthylate de sodium dans le méthanol. Par dilution du milieu réactionnel à l'eau, on précipite le produit attendu. Après essorage, lavage à l'eau et séchage sous vide en présence de $P_2O_5$, le produit obtenu est recristallisé du toluène ; un insoluble noir est éliminé par filtration à chaud. Il fond à 140°C.

L'analyse du produit obtenu donne les résultats suivants :

| Analyse | Calculé pour $C_7H_9N_3O_3$ | Trouvé |
|---|---|---|
| C% | 45,90 | 45,86 |
| H% | 4,96 | 4,91 |
| N% | 22,95 | 22,87 |
| O% | 26,23 | 26,52 |

Exemple de préparation 2

Préparation du 2-(β-hydroxyéthyl) amino-6-(β-hydroxyéthyl) amino-4-méthoxynitrobenzène

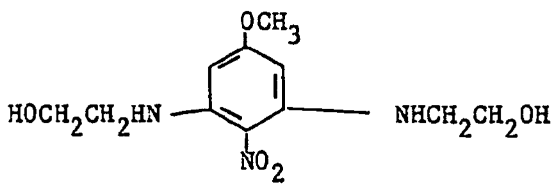

1ère étape

Préparation du 2-(β-hydroxyéthyl) amino-6-(β-hydroxyéthyl) amino-4-chloronitrobenzène

0,132 mole (30 g) de 2,4,6-trichloronitrobenzène sont chauffés à 95°C dans 120 ml d'éthanolamine. Au bout de 30 minutes, le milieu réactionnel est versé sur 240 g d'un mélange glace-eau. Le produit attendu précipite. Il est essoré, lavé à l'eau puis séché sous vide en présence de pentaoxyde de phosphore. Après recristallisation de l'éthanol absolu, il fond à 154°C.

L'analyse du produit obtenu donne les résultats suivants :

| Analyse | Calculé pour $C_{10}H_{14}ClN_3O_4$ | Trouvé |
|---|---|---|
| C% | 43,56 | 43,37 |
| H% | 5,08 | 5,11 |
| N% | 15,24 | 15,25 |
| O% | 23,23 | 23,45 |
| Cl% | 12,88 | 13,01 |

2ème étape :

Préparation du 2-(β-hydroxyéthyl) amino-6-(β-hydroxyéthyl)amino-4-méthoxynitrobenzène

On chauffe au bain marie bouillant 0,09 mole (25 g) de 2-(β-hydroxyéthyl) amino-6-(β-hydroxyéthyl) amino-4-chloro-nitrobenzène dans 100 ml d'une solution à 25% de méthylate de sodium dans le méthanol. Après 1 heure 30 minutes de chauffe, le produit attendu est précipité du milieu réactionnel par ajout de 250 g d'eau glacée. Après essorage et séchage à chaud sous vide en présente ce $P_2O_5$, le produit obtenu est recristallisé de l'éthanol à 96°. Il fond à 150°C.

L'analyse du produit obtenu donne les résultats suivants :

| Analyse | Calculé pour $C_{11}H_{17}N_3O_5$ | Trouvé |
|---|---|---|
| C% | 48,70 | 48,91 |
| H% | 6,32 | 6,41 |
| N% | 15,49 | 15,61 |
| O% | 29,49 | 29,30 |

Exemple de préparation 3

Préparation du 2-[(3-méthylamino-5-méthylamino-4-nitro) phénoxy] éthanol

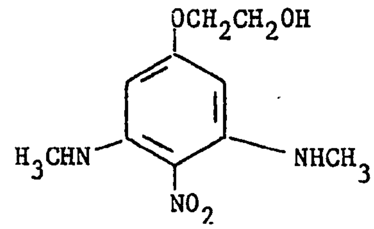

1ère étape

Préparation du 4-chloro-2-méthylamino-6-méthylaminonitrobenzène

A 300 ml d'une solution de méthylamine à 30% dans l'éthanol absolu on ajoute par portions, à température ambiante, 0,150 mole (34 g) de 2,4,6-trichloronitrobenzène. Après 23 heures sous agitation à température ambiante, on filtre le précipité constitué essentiellement de 4-chloro-2-méthylamino-6-méthylaminonitrobenzène, chromatographiquement pur et qui fond à 193°C.

2ème étape

Préparation du 2-[(3-méthylamino-5-méthylamino-4-nitro) phénoxy] éthanol

On ajoute par portions 0,131 mole (28,3 g) de 4-chloro-2-méthylamino-6-méthylaminonitrobenzène à une solution préparée à 50°C, de 17, 2 g de potasse en pastilles dans 66 ml d'éthylèneglycol et 100 ml de N-méthylpyrrolidone. On chauffe 3 heures au bain-marie bouillant. Par ajout de 300 g d'eau glacée, on précipite le produit attendu. Après essorage puis séchage sous vide en présence de $P_2O_5$, il est recristallisé de l'éthanol à 96°. Il fond à 142°C.

L'analyse du produit obtenu donne les résultats suivants :

| Analyse | Calculé pour $C_{10}H_{15}N_3O_4$ | Trouvé |
|---|---|---|
| C% | 49,78 | 49,73 |
| H% | 6,27 | 6,28 |
| N% | 17,42 | 17,38 |
| O% | 26,53 | 26,64 |

Exemple de préparation 4

Préparation du 3-[(3-méthylamino-5-méthylamino-4-nitro) phénoxy] propane 1,2-diol

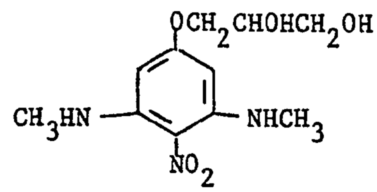

1ère étape :

Préparation du 2-méthylamino-6-méthylamino 4-fluoronitrobenzène

On introduit, goutte à goutte, 11 ml de 2,4,6-trifluoronitrobenzène à 65 ml d'une solution de méthylamine

à 25% dans l'éthanol absolu, la température est maintenue entre −10°C et 0°C par refroidissement. Lorsque l'addition est terminée, le milieu réactionnel est abandonné à température ambiante 1 heure ; le produit attendu qui a cristallisé du milieu réactionnel est essoré, lavé puis séché à chaud sous vide en présence de $P_2O_5$. Il fond à 198°C.

L'analyse du produit obtenu donne les résultats suivants :

| Analyse | Calculé pour $C_8H_{10}N_3O_2F$ | Trouvé |
|---|---|---|
| C% | 48,24 | 48,13 |
| H% | 5,02 | 5,03 |
| N% | 21,10 | 21,02 |
| F% | 9,55 | 9,62 |

2ème étape :

Préparation du 3-[(3-méthylamino-5-méthylamino-4-nitro) phénoxy] propane 1,2-diol

On ajoute une solution préparée à 90°C, de 880 mg de soude en pastilles dans 8 g de glycérol additionné de 0,3 ml d'eau, à une solution de 0,01 mole de 2-méthylamino-6-méthylamino-4-fluoronitrobenzène dans 8 g de glycérol préalablement chauffée à 90°C. Après 2 heures de chauffage au bain-marie bouillant, le milieu réactionnel est chauffé 20 minutes à 130°C. Le produit attendu précipite du milieu réactionnel après dilution de celui-ci par 20 ml d'eau glacée. Après séchage sous vide, il est recristallisé de l'isopropanol, puis lavé à l'acétate d'éthyle au reflux ; il fond à 155°C.

L'analyse du produit obtenu donne les résultats suivants :

| Analyse | Calculé pour $C_{11}H_{17}N_3O_5$ | Trouvé |
|---|---|---|
| C% | 48,71 | 48,84 |
| H% | 6,27 | 6,39 |
| N% | 15,50 | 15,40 |
| O% | 29,52 | 29,39 |

Exemple de préparation 5

Préparation du 2,6-diamino-4-hydroxynitrobenzène

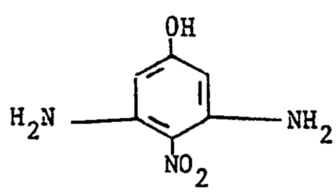

1 g de 2,6-diamino-4-méthoxynitrobenzène préparé à l'exemple 1 est chauffé 15 minutes au bain-marie bouillant dans 3 ml d'acide bromhydrique à 66%. Par ajout d'eau, le produit attendu précipite sous forme de bromhydrate. Le bromhydrate est mis en suspension dans l'eau puis dissous par ajout de soude concentrée afin d'éliminer un insoluble par filtration en présence de noir animal. Le produit attendu précipite du filtrat par ajout d'acide chlorhydrique concentré. Après essorage puis séchage sous vide en présence de $P_2O_5$, il se décompose à 264°C.

L'analyse du produit attendu donne les résultats suivants :

| Analyse | Calculé pour $C_6H_7N_3O_3$ | Trouvé |
|---|---|---|
| C% | 42,60 | 42,51 |
| H% | 4,17 | 4,22 |
| N% | 24,85 | 24,73 |
| O% | 28,38 | 28,28 |

Exemple de préparation 6

Préparation du 2-méthylamino-6-méthylamino-4-(β-hydroxypropyl)amino-nitrobenzène

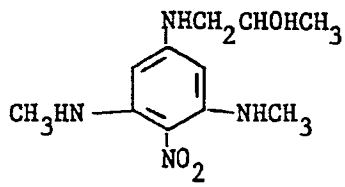

On porte à 100°C, sous agitation 0,01 mole (2g) de 2-méthylamino-6-méthylamino-4-fluoronitrobenzène dans 8 ml de 1-amino-2-propanol. Après 15 minutes de chauffage, le milieu réactionnel est dilué par 10 ml d'eau et neutralisé par ajout d'acide chlorhydrique. Le précipité du produit attendu est essoré. Après séchage, il est recristallisé de l'éthanol à 96°. Il fond à 231°C.

L'analyse du produit obtenu donne les résultats suivants :

| Analyse | Calculé pour $C_{11}H_{18}N_4O_3$ | Trouvé |
|---|---|---|
| C% | 51,97 | 52,02 |
| H% | 7,09 | 7,16 |
| N% | 22,05 | 21,92 |
| O% | 18,90 | 19,07 |

Exemple de préparation 7

Préparation du 2-méthylamino-6-méthylamino-4-(β, γ-dihydroxypropyl)amino-nitrobenzène

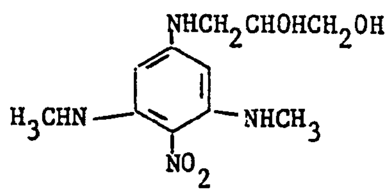

On chauffe au reflux pendant 15 heures le mélange constitué de 0,01 mole (2 g) de 2-méthylamino-6-méthylamino-4-fluoro-nitrobenzène et de 3,8 g de 3-amino-propane-1,2-diol dans 7 ml de dioxane additionnés de 1 ml de N-méthylpyrrolidone.

Le milieu réactionnel est dilué par 20 ml d'eau glacée. Le produit attendu précipite. Après lavage et séchage sous vide en présence de $P_2O_5$, il est recristallisé de l'éthanol à 96°. Il fond à 210°C.

L'analyse du produit obtenu donne les résultats suivants :

| Analyse | Calculé pour $C_{11}H_{18}N_4O_4$ | Trouvé |
|---|---|---|
| C% | 48,89 | 48,94 |
| H% | 6,67 | 6,59 |
| N% | 20,74 | 20,67 |
| O% | 23,70 | 23,53 |

Exemple de préparation 8

Préparation du 2-méthylamino-6-méthylamino-4-[(β-hydroxyéthoxy)éthyl] amino-nitrobenzène

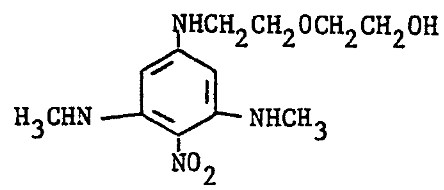

On chauffe à 100°C 0,01 mole (2 g) de 2-méthylamino-6-méthylamino-4-fluoro-nitrobenzène dans 8 ml de 2-(2-aminoéthoxy) éthanol. Après 30 minutes, le milieu réactionnel est dilué par 20 ml d'eau glacée. Le produit attendu précipite ; après essorage puis séchage en présence de $P_2O_5$ sous vide, il est recristallisé de l'isopropanol puis de l'acétate d'éthyle ; il fond à 132°C.

L'analyse du produit obtenu donne les résultats suivants :

| Analyse | Calculé pour $C_{12}H_{20}N_4O_4$ | Trouvé |
|---|---|---|
| C% | 50,69 | 50,65 |
| H% | 7,09 | 7,08 |
| N% | 19,71 | 19,65 |
| O% | 22,51 | 22,81 |

Exemple de préparation 9

Préparation du 2-méthylamino-6-(β-méthoxyéthyl) amino-4-méthoxynitrobenzène

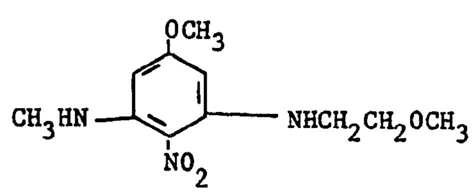

1ère étape :

Préparation du 2-méthylamino-4,6-dichloronitrobenzène

On opère de la même façon que dans l'exemple 3 (1ère étape). Après avoir filtré le précipité constitué de 4-chloro-2-méthylamino-6-méthylamino-nitrobenzène, on recueille le filtrat qui est évaporé à sec sous pression réduite. A l'extrait sec ainsi obtenu on ajoute 800 ml d'acide chlorhydrique concentré. La fraction insoluble est éliminée par essorage. Après dilution du filtrat par 650 ml d'eau, le produit attendu précipite. Il est dilué à l'eau puis séché sous vide en présence de pentaoxyde de phosphore. Après recristallisation de l'isopropanol puis de l'éthanol absolu, il fond à 120°C

L'analyse du produit obtenu donne les résultats suivants :

| Analyse | Calculé pour $C_7H_6Cl_2N_2O_2$ | Trouvé |
| --- | --- | --- |
| C% | 38,01 | 38,02 |
| H% | 2,71 | 2,72 |
| N% | 12,67 | 12,77 |
| O% | 14,48 | 14,40 |
| Cl% | 32,13 | 32,01 |

2ème étape

Préparation du 2-méthylamino-6-(β-méthoxyéthyl) amino-4-chloronitrobenzène

On ajoute par portions 0,09 mole (20 g) de 2-méthylamino-4,6-dichloronitrobenzène à 80 ml de 2-méthoxyéthylamine préalablement chauffés à 80°C. Après 2 heures de chauffage, le milieu réactionnel est dilué par 100 ml d'eau glacée. Le produit attendu précipite. Après essorage, lavage à l'eau puis séchage sous vide en présence de $P_2O_5$, il est recristallisé de l'éthanol à 96°. Il fond à 93°C.

L'analyse du produit obtenu donne les résultats suivants :

| Analyse | Calculé pour $C_{10}H_{14}ClN_3O_3$ | Trouvé |
| --- | --- | --- |
| C% | 46,24 | 46,25 |
| H% | 5,40 | 5,35 |
| N% | 16,18 | 16,24 |
| O% | 18,50 | 18,61 |
| Cl% | 13,68 | 13,52 |

3ème étape

Préparation du 2-méthylamino-6-(β-méthoxyéthyl) amino-4-méthoxynitrobenzène

On porte au reflux 0,019 mole (5 g) de 2-méthylamino 6-(β-méthoxyéthyl) amino-4-chloro-nitrobenzène dans 35 ml d'une solution de méthylate de sodium à 26% dans le méthanol. Le milieu réactionnel, après 45 minutes de chauffage est dilué par 100 ml d'eau glacée ; le produit attendu précipite. Après essorage, lavage à l'eau puis séchage sous vide en présence de $P_2O_5$, le produit obtenu est recristallisé de l'isopropanol. Il fond à 84°C.

L'analyse du produit obtenu donne les résultats suivants :

| Analyse | Calculé pour $C_{11}H_{17}N_3O_4$ | Trouvé |
| --- | --- | --- |
| C% | 51,76 | 51,75 |
| H% | 6,67 | 6,71 |
| N% | 16,47 | 16,46 |
| O% | 25,10 | 24,99 |

Exemple de préparation 10

Préparation du 2-(β-hydroxyéthyl) amino-6-(β-hydroxyéthyl) amino-4-(β-aminoéthyl) aminonitrobenzène

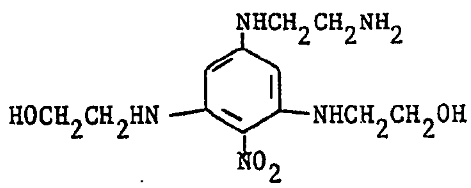

On chauffe à 130°C pendant 2 heures 0,13 mole (36,5 g) de 2-(β-hydroxyéthyl) amino-6-(β-hydroxyéthyl) amino-4-chloronitrobenzène dont la synthèse est décrite à la 1ère étape de l'exemple 2 ci-dessus, dans 150 ml d'éthylènediamine. Le milieu réactionnel est dilué par 450 ml d'eau glacée. Le produit attendu précipite. Après essorage, lavage à l'eau jusqu'à neutralité, le produit obtenu est séché sous vide en présence de $P_2O_5$. Il est recristallisé de l'éthanol à 96°. Il fond à 220°C.

L'analyse du produit obtenu donne les résultats suivants :

| Analyse | Calculé pour $C_{12}H_{21}N_5O_4$ | Trouvé |
|---|---|---|
| C% | 48,16 | 48,14 |
| H% | 7,02 | 7,07 |
| N% | 23,41 | 23,26 |
| O% | 21,40 | 21,62 |

Exemple de préparation 11

Préparation du 2-(β-hydroxyéthyl) amino-4-(β-hydroxyéthyl) amino-6-(β-hydroxyéthyl) amino-nitrobenzène

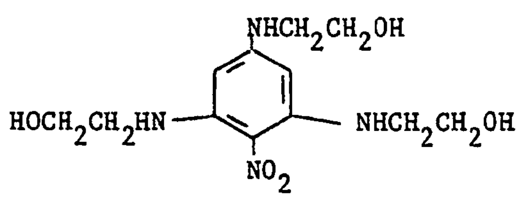

On chauffe à 100-110°C, 0,0072 mole (2 g) de 2-(β-hydroxyéthyl)amino-6-(β-hydroxyéthyl) amino-4-chloronitrobenzène, synthétisé à la 1ère étape de l'exemple 2, dans 6 ml d'éthanolamine pendant 6 heures. Le mélange réactionnel est dilué à l'eau glacée, puis amené à neutralité par ajout d'acide chlorhydrique concentré ; le produit attendu précipite. Après essorage et séchage à chaud sous vide en présence de $P_2O_5$, il est recristallisé de l'éthanol à 96°. Il fond à 200°C.

L'analyse du produit obtenu donne les résultats suivants :

| Analyse | Calculé pour $C_{12}H_{20}N_4O_5$ | Trouvé |
|---|---|---|
| C% | 48,00 | 47,95 |
| H% | 6,67 | 6,50 |
| N% | 18,67 | 18,64 |
| O% | 26,66 | 26,78 |

Exemple de préparation 12

Préparation du [3-(β-hydroxyéthyl) amino-5-(β-hydroxyéthyl) amino-4-nitrophényl]-β-hydroxyéthylthioéther

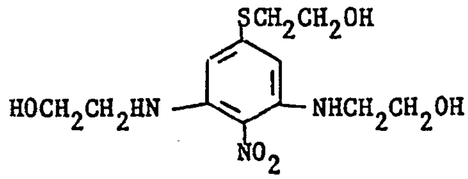

A 0,08 mole (22 g) de 2-(β-hydroxyéthyl) amino-6-(β-hydroxyéthyl) amino-4-chloronitrobenzène dont la synthèse est décrite à la 1ère étape de l'exemple 2, dans 44 ml d'éthanol absolu, on ajoute goutte à goutte une solution constituée de 7,5 g de potasse en pastilles dissoute dans 9,5 ml de thioéthanol. Le milieu réactionnel est réactionnel est chauffé 2 heures 30 minutes au reflux. Après refroidissement, le produit attendu précipite. Il est essoré, puis réempâté dans l'eau. Après séchage, il est recristallisé de l'éthanol. Il fond à 157°C.

L'analyse du produit obtenu donne les résultats suivants :

| Analyse | Calculé pour $C_{12}H_{19}N_3SO_5$ | Trouvé |
|---|---|---|
| C% | 45,42 | 45,21 |
| H% | 5,99 | 5,99 |
| N% | 13,25 | 13,18 |
| O% | 25,24 | 25,29 |
| S% | 10,10 | 9,99 |

Exemple de préparation 13

Préparation du 2-(γ-hydroxypropyl) amino-4-(γ-hydroxypropyl) amino-6-(γ-hydroxypropyl) amino-nitrobenzène

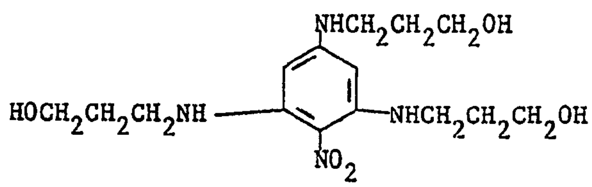

En opérant de la même manière que dans l'exemple 11 on obtient, en remplaçant l'éthanolamine par la propanolamine, le produit attendu qui est recristallisé de l'éthanol ; il fond à 150°C.

L'analyse du produit obtenu donne les résultats suivants :

| Analyse | Calculé pour $C_{15}H_{26}N_4O_5$ | Trouvé |
|---|---|---|
| C% | 52,62 | 52,68 |
| H% | 7,65 | 7,66 |
| N% | 16,36 | 16,22 |
| O% | 23,37 | 23,37 |

Exemple de préparation n°14

Préparation du 4-β-méthoxyéthoxy-2-(β-hydroxyéthyl) amino-6-(β-hydroxyéthyl) amino nitrobenzène

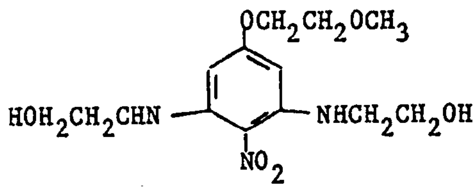

On ajoute 0,09 mole (25 g) de 4-chloro-2-(β-hydroxyéthyl) amino-6-(β-hydroxyéthyl) amino nitrobenzène synthétisé à la 1ère étape de l'exemple 2 à une solution préparée à 80°C de 11, 9 g de potasse en pastilles dans 60 ml de 2-méthoxyéthanol et 25 ml de N-méthylpyrrolidone. On chauffe 4 heures au bain-marie bouillant. Le mélange réactionnel est dilué par 350 ml d'eau glacée. Le produit attendu est obtenu par chromatographie sous pression. Environ 150 ml de la solution aqueuse obtenue par dilution du milieu réactionnel sont injectés sur une colonne chromatographique $C_{18}$RD (Appareillage Prep 500 Waters). Le produit attendu est élué par une solution de 40% de méthanol et 60% d'eau. Après évaporation des fractions contenant le produit attendu, on obtient un extrait sec que l'on recristallise de l'isopropanol. Après recristallisation, il fond à 116°C.

Exemple de préparation n°15

Préparation du 2-(β, γ-dihydroxypropyl) amino-6-(β, γ-dihydroxypropyl) amino-4-méthoxynitrobenzène

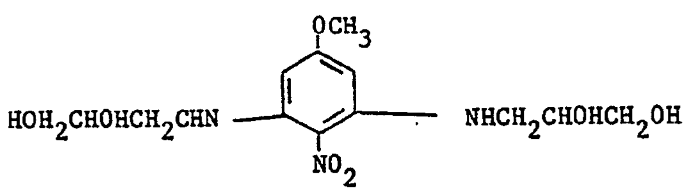

1ère étape

Préparation du 4-chloro-2-(β, γ-dihydroxypropyl) amino-6-(β, γ-dihydroxypropyl) aminonitrobenzène

On chauffe au reflux le mélange constitué par 0,1 mole (22,6 g) de 2,4,6-trichloronitrobenzène et de 54,7 g de 3-amino-1,2-propanediol dans 20 ml de dioxane. Après 4 heures de chauffage, on évapore le dioxane sous pression réduite. L'huile obtenue est diluée dans environ 300 ml d'eau. Le produit attendu est obtenu par chromatographie sous pression en deux opérations. Environ 200 ml de la solution aqueuse du produit attendu contenant le 3-amino-1,2-propanediol sont injectés sur une colonne chromatographique $C_{18}$RD (Appareillage Prep 500 Waters). Le produit attendu est élué par une solution de 35% de méthanol et 65% d'eau. Après évaporation des fractions contenant le produit attendu, on obtient un extrait sec que l'on recristallise de l'alcool à 96°.

Le produit obtenu fond à 146°C.

L'analyse élémentaire du produit obtenu donne les résultats suivants :

| Analyse | Calculé pour $C_{12}H_{18}N_3O_6Cl$ | Trouvé |
|---|---|---|
| C% | 42,92 | 42,87 |
| H% | 5,36 | 5,37 |
| N% | 12,52 | 12,39 |
| O% | 28,61 | 28,69 |
| Cl% | 10,58 | 10,47 |

2ème étape

Préparation du 2-(β, γ-dihydroxypropyl) amino-6-(β, γ-dihydroxypropyl) amino-4-méthoxynitrobenzène

On chauffe 7 heures au reflux 4,56 g du composé préparé à l'étape précédente dans 30 ml d'une solution à 20% de méthylate de sodium dans le méthanol. Après dilution du milieu réactionnel à l'eau puis acidification, le méthanol est éliminé par évaporation sous pression réduite. Après extraction du résidu d'évaporation de l'acétate d'éthyle, on obtient un extrait sec que l'on purifie par chromatographie sous pression (Appareillage Prep 500 Waters ; Colonne $C_{18}$RD ; éluant : méthanol 30%, eau 70%). Le produit attendu fond à 130°C.

Exemple de préparation n°16

Préparation du 2-amino-6-(β-hydroxyéthyl) amino-4-méthoxynitrobenzène

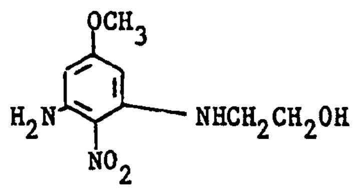

1ère étape

Préparation du 2-amino-6-(β-hydroxyéthyl) amino-4-chloronitrobenzène

On chauffe 2 heures au bain-marie bouillant 0,05 mole (11,2 g) de 2-amino-4,6-dichloronitrobenzène (Recueil Trav. Chim. 68 p 88 [1949]) dans 45 ml d'éthanolamine. Le milieu réactionnel est dilué par 150 ml d'eau glacée. Après acidification par 80 ml d'acide chlorhydrique, le produit attendu précipite. Après recristallisation de 400 ml d'isopropanol, il fond à 183°C. L'analyse du produit obtenu donne les résultats suivants:

| Analyse | Calculé pour $C_8H_{10}N_3O_3Cl$ | Trouvé |
|---|---|---|
| C% | 41,47 | 41,42 |
| H% | 4,32 | 4,10 |
| N% | 18,14 | 17,98 |
| O% | 20,73 | 20,53 |
| Cl% | 15,33 | 15,18 |

2ème étape

Préparation du 2-amino-6-(β-hydroxyéthyl) amino-4-méthoxynitrobenzène

On chauffe 2 heures 30 au bain-marie bouillant 0,172 mole (40 g) de 2-amino-6(β-hydroxyéthyl) amino 4-chloro-nitrobenzène dans 160 ml d'une solution à 30% de méthylate de sodium dans le méthanol. Le produit attendu précipite par dilution avec 350 ml d'eau glacée. Après recristallisation de l'isopropanol bouillant afin d'éliminer un insoluble, le produit attendu fond à 148°C.

L'analyse du produit obtenu donne les résultats suivants :

| Analyse | Calculé pour $C_9H_{13}N_3O_4$ | Trouvé |
|---|---|---|
| C% | 47,58 | 47,37 |
| H% | 5,73 | 5,77 |
| N% | 18,51 | 18,41 |
| O% | 28,20 | 28,26 |

Exemple d'application 1

On prépare le mélange tinctorial suivant :

| | | | |
|---|---|---|---|
| - 2-(β-hydroxyéthyl)amino-6-(β-hydroxyéthyl)amino-4-(β-aminoéthyl)aminonitrobenzène | | | 0,125 g |
| - 2-butoxyéthanol | | | 8 g |
| - COMPERLAN KD - Société HENKEL (diéthanolamide d'acide gras de coprah) | | | 2,2 g |
| - Acide laurique | | | 0,8 g |
| - Monoéther éthylique de l'éthylène glycol | | | 2 g |
| - Monoéthanolamine | qs | pH : 10 | |
| - Eau | qsp | | 100 g |

Ce mélange, appliqué 25 minutes à 35°C sur cheveux naturellement blancs à 90%, leur confère, après shampooing et rinçage, une coloration beige doré pâle.

Exemple d'application 2

On prépare le mélange tinctorial suivant :

| | | | |
|---|---|---|---|
| - 2-(γ-hydroxypropyl)amino 4-(γ-hydroxypropyl)-amino-6-(γ-hydroxypropyl)aminonitrobenzène | | | 0,067 g |
| - Alcool à 96° | | | 10 g |
| - Carbopol 934 - Société GOODRICH CHEMICALS (acide polyacrylique réticulé) | | | 2 g |
| - Triéthanolamine | qs | pH : 8 | |
| - Eau | qsp | | 100 g |

Ce mélange, appliqué 28 minutes à 35°C sur cheveux décolorés, leur confère, après shampooing et rinçage, une coloration abricot.

Exemple d'application 3

On prépare le mélange tinctorial suivant :

| | | | |
|---|---|---|---|
| - 2-méthylamino-6-(β-méthoxyéthyl)amino-4-méthoxynitrobenzène | | | 0,124 g |
| - CELLOSIZE W.P. 03 - Société UNION CARBIDE (hydroxyéthylcellulose) | | | 2 g |
| - Lauryl sulfate d'ammonium | | | 5 g |
| - Monoéthanolamine | qs | pH : 10 | |
| - Eau | qsp | | 100 g |

Ce mélange, appliqué 30 minutes à 35°C sur cheveux décolorés, leur confère, après shampooing et rinçage, une coloration rose-orangé.

Exemple d'application 4

On prépare le mélange tinctorial suivant :

| | | | |
|---|---|---|---|
| - 2,6-diamino-4-hydroxynitrobenzène | | | 0,108 g |
| - Propylèneglycol | | | 12 g |
| - CARBOPOL 934 - Société GOODRICH CHEMICALS (acide polyacrylique réticulé) | | | 2 g |
| - Monoéthanolamine | qs | pH : 9 | |
| - Eau | qsp | | 100 g |

Ce mélange, appliqué 25 minutes à 35°C sur cheveux décolorés, leur confère, après shampooing et rinçage, une coloration beige paille.

Exemple d'application 5

On prépare le mélange tinctorial suivant :

| | | | |
|---|---|---|---|
| - 2-méthylamino-6-méthylamino-4-[(β-hydroxyéthoxy)-éthyl]aminonitrobenzène | | | 0,094 g |
| - Alcool à 96° | | | 12 g |
| - CELLOSIZE W.P. 03 - Société UNION CARBIDE (hydroxyéthylcellulose) | | | 2 g |
| - Chlorure de cétyl diméthyl hydroxyéthyl ammonium | | | 2 g |
| - Monoéthanolamine | qs | pH : 11 | |
| - Eau | qsp | | 100 g |

Ce mélange, appliqué 25 minutes à 35°C sur cheveux permanentés, leur confère, après shampooing et rinçage, une coloration beige orangé.

Exemple d'application 6

On prépare le mélange tinctorial suivant :

| | | | |
|---|---|---|---|
| - 2-(β-hydroxyéthyl)amino-6-(β-hydroxyéthyl)amino-4-(β-aminoéthyl)aminonitrobenzène | | | 0,1 g |
| - Chlorhydrate de 2-(β-aminoéthyl)amino-4-méthoxynitrobenzène | | | 0,05 g |
| - Chlorhydrate de 5-N,N di(β -hydroxyéthyl)amino-2-(β-aminoéthyl)aminonitrobenzène | | | 0,043 g |
| - 2-butoxyéthanol | | | 10 g |
| - ALFOL C 16/18 - Société CONDEA (alcool cétylstéarylique) | | | 8 g |
| - CIRE DE LANETTE E - Société HENKEL (sulfate cétylstéarylique de sodium) | | | 0,5 g |
| - CEMULSOL B - Société RHONE POULENC (huile de ricin éthoxylée) | | | 1 g |
| - Diéthanolamide oléique | | | 1,5 g |
| - Triéthanolamine | qs | pH 8,5 | |
| - Eau | qsp | | 100 g |

Ce mélange, appliqué 30 minutes à 35°C sur cheveux naturellement blancs à 90%, leur confère, après shampooing et rinçage, une coloration châtain très clair doré.

<u>Exemple d'application 7</u>

On prépare le mélange tinctorial suivant :

| | | | |
|---|---|---|---|
| - [β-(β-hydroxyéthyl)amino-5-(β-hydroxyéthyl)-amino-4-nitrophényl]-β-hydroxyéthylthioéther | | | 0,1 g |
| - 2-butoxyéthanol | | | 10 g |
| - LAURAMIDE - Société WITCO (monoéthanolamide d'acide laurique) | | | 1,5 g |
| - Acide laurique | | | 1 g |
| - CELLOSIZE W.P. 03 - Société UNION CARBIDE (hydroxyéthylcellulose) | | | 5 g |
| - Monoéthanolamine | qs | pH : 9 | |
| - Eau | qsp | | 100 g |

Ce mélange, appliqué 25 minutes à 35°C sur cheveux décolorés, leur confère, après shampooing et rinçage, une coloration rose pâle.

<u>Exemple d'application 8</u>

On prépare le mélange tinctorial suivant :

| | | | |
|---|---|---|---|
| - 2-(β-hydroxyéthyl)amino-6-(β-hydroxyéthyl)amino-4-méthoxynitrobenzène | | | 0,105 g |
| - 2,4-di(γ-hydroxypropyl)amino-5-chloro-nitrobenzène | | | 0,064 g |
| - 5-N,N-di(β-hydroxyéthyl)amino-2-(β-hydroxyéthyl)-aminonitrobenzène | | | 0,035 g |
| - Alcool à 96° | | | 10 g |
| - CEMULSOL NP 4 - Société RHONE POULENC (nonylphénol à 4 moles O.E) | | | 12 g |
| - CEMULSOL NP 9 - Société RHONE POULENC (nonylphénol à 9 moles O.E) | | | 15 g |
| - Alcool oléique polyglycérolé à 2 moles de glycérol | | | 1,5 g |
| - Alcool oléique polyglycérolé à 4 moles de glycérol | | | 1,5 g |
| - Monoéthanolamine | qs | pH : 9 | |
| - Eau | qsp | | 100 g |

Ce mélange, appliqué 25 minutes à 35°C sur cheveux décolorés, leur confère, après shampooing et rinçage, une coloration beige doré.

Exemple d'application 9

Teinture d'oxydation

On prépare le mélange tinctorial suivant :

| | | | |
|---|---|---|---|
| - 2-amino-6-(β-hydroxyéthyl)amino-4-méthoxy-nitrobenzène | | 1,5 | g |
| - Paraphénylènediamine | | 0,1 | g |
| - Paraminophénol | | 0,07 | g |
| - Métaaminophénol | | 0,17 | g |
| - Dichlorhydrate de (2,4-diamino)phénoxyéthanol | | 0,06 | g |
| - Hémisulfate de 4-N-méthylaminophénol | | 0,15 | g |
| - ALFOL C 16/18 - Société CONDEA (alcool cétylstéarylique) | | 8 | g |
| - CIRE DE LANETTE E - Société HENKEL (sulfate cétylstéarylique de sodium) | | 0,5 | g |
| - CEMULSOL B - RHONE POULENC (huile de ricin éthoxylée) | | 1 | g |
| - Diéthanolamide oléique | | 1,5 | g |
| - MASQUOL DTPA - Société PROTEX (sel pentasodique de l'acide diéthylène triamine pentacétique) | | 2,5 | g |
| - Ammoniaque 22°Bé | | 11 | g |
| - Eau | qsp | 100 | g |
| - pH 10,3 | | | |

Au moment de l'emploi, on ajoute 100 g d'eau oxygénée à 20 volumes. Le mélange, appliqué 25 minutes à 38°C sur des cheveux décolorés, leur confère, après shampooing et rinçage, une coloration châtain violine.

## Revendications

### Revendications pour les Etats Contractants : AT, BE, CH, DE, FR, GB, GR, IT, LI, NL, SE

1. 2-nitro métaphénylènediamine substituée de formule :

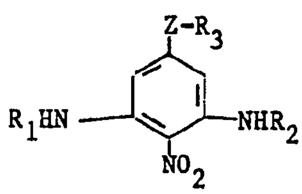

(I)

dans laquelle Z désigne le radical -O-, -S- ou -NH- et $R_1$, $R_2$, et $R_3$, identiques ou différents désignent un atome d'hydrogène, un radical alkyle, hydroxyalkyle, polyhydroxyalkyle, alcoxyalkyle, hydroxyalcoxyalkyle ou aminoalkyle dont le radical amino peut être mono- ou disubstitué par un radical alkyle ou hydroxyalkyle, les radicaux alkyle et alcoxy comportant 1 à 6 atomes de carbone, ou les sels cosmétiquement acceptables de ce composé.

2. Composé selon la revendication 1, caractérisé par le fait que $R_1$, $R_2$ et $R_3$ identiques ou différents, désignent un atome d'hydrogène, un radical méthyle, éthyle, n-propyle, n-butyle, ß-hydroxyéthyle, β-hydroxypropyle, γ-hydroxypropyle, β, γ-dihydroxypropyle, méthoxyéthyle, éthoxyéthyle, ß-hydroxyéthoxyéthyle, ß-aminoéthyle, β-hydroxyéthylaminoéthyle ou β-diéthylaminoéthyle.

3. Composé selon la revendication 1 ou 2, caractérisé par le fait qu'il est choisi parmi le groupe comprenant le 2,6-diamino-4-méthoxynitrobenzène, le 2,6-diamino-4-hydroxynitrobenzène, le 2-(β-hydroxyéthyl)

amino-6-(β-hydroxyéthyl) amino-4-méthoxynitrobenzène, le 2-[(3-méthylamino-5-méthylamino-4-nitro) phénoxy] éthanol, le 3-[(3-méthylamino-5-méthylamino-4-nitro) phénoxy] propane 1,2-diol, le 2-méthylamino-6-(β-méthoxyéthyl) amino-4-méthoxynitrobenzène, le 4-β-méthoxyéthoxy-2-(β-hydroxyéthyl) amino-6-(β-hydroxyéthyl)aminonitrobenzène, le 2-méthylamino-6-méthylamino-4-(β-hydroxypropyl) aminonitrobenzène, le 2-méthylamino-6- méthylamino-4-(β, γ -dihydroxypropyl) aminonitrobenzène, le 2-méthylamino-6-méthylamino-4-[(β-hydroxyéthoxy) éthylamino] nitrobenzène, le 2-(β-hydroxyéthyl)amino-6-(β-hydroxyéthyl) amino-4-(β-aminoéthyl) aminonitrobenzène, le 2-(β-hydroxyéthyl) amino-4-(β-hydroxyéthyl) amino-6-(β-hydroxyéthyl) aminonitrobenzène, le [3-(β-hydroxyéthyl) amino-5-(β-hydroxyéthyl) amino-4-nitrophényl] β-hydroxyéthylthioéther, le 2-(β, γ -dihydroxypropyl) amino-6-(β, γ-dihydroxypropyl) amino-4-méthoxynitrobenzène, le 2-(γ-hydroxypropyl) amino-4-(γ-hydroxypropyl)amino-6-(γ-hydroxypropyl) aminonitrobenzène et le 2-amino-6-(β-hydroxyéthyl) amino-4-méthoxynitrobenzène.

4. Procédé de préparation d'une 2-nitro métaphénylènediamine substituée de formule (I) selon la revendication 1, caractérisé par le fait qu'il consiste à disubstituer un 2,4,6-trihalogénonitrobenzène de formule (II) :

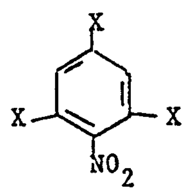

(II)

dans laquelle X représente un atome d'halogène, et en particulier le chlore ou le fluor pour obtenir un 2,6-diamino-4-halogénonitrobenzène de formule (IV) :

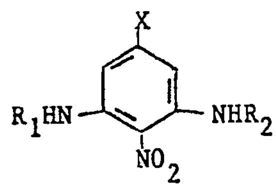

(IV)

dans laquelle $R_1$ et $R_2$ ont les significations indiquées dans la revendication 1, éventuellement en présence de solvant et à des températures variant entre −10°C et la température de reflux de l'amine $R_1NH_2$ et/ou $R_2NH_2$ utilisée pour la substitution ou de celle du solvant, puis à faire réagir le composé de formule (IV) avec un composé de formule $HZR_3$, en présence ou non de solvant, à une température comprise entre 30 et 150°C.

5. Procédé de préparation du composé de formule (I) selon la revendication 4, caractérisé par le fait que le composé de formule (IV) dans lequel $R_1$ et $R_2$ ont des significations différentes est préparé en faisant réagir dans un premier temps, l'ammoniac ou une amine de formule $R_1NH_2$, $R_1$ ayant la signification ci-dessus définie, sur le 2,4,6-trihalogénonitrobenzène de formule (II) pour conduire au composé de formule (III):

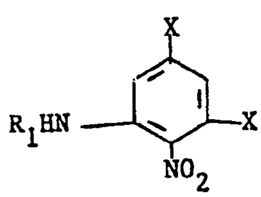

(III)

qui est soumis à l'action d'une amine $R_2NH_2$ ou d'ammoniac pour conduire au composé de formule (IV).

6. Procédé de préparation du composé de formule (I) selon la revendication 4, caractérisé par le fait que le composé (IV) dans lequel le groupement $R_2$ est identique au groupement $R_1$ et différent d'un atome d'hydrogène est préparé en une seule étape à partir du composé (II) par réaction de ce composé avec une amine de formule $R_1NH_2$.

7. Procédé de préparation du composé de formule (I) selon la revendication 1, dans laquelle $R_1$ et $R_2$ désignent H ou alkyle, $R_3$ = H et Z = 0, caractérisé par le fait que l'on soumet un composé de formule (I) dans laquelle Z = 0 et $R_3$ = $CH_3$ à l'action de l'acide bromhydrique à une température comprise entre 50 et

100°C.

8. Composition tinctoriale pour fibres kératiniques, et en particulier pour cheveux humains, caractérisée par le fait qu'elle contient, dans un milieu solvant, au moins une 2-nitro métaphénylènediamine substituée de formule (I) selon la revendication 1, ou un sel cosmétiquement acceptable de ce composé.

9. Composition tinctoriale selon la revendication 8, caractérisée par le fait qu'elle contient, dans un milieu solvant, au moins un composé choisi parmi le groupe comprenant le 2,6-diamino-4-méthoxynitrobenzène, le 2,6-diamino-4-hydroxynitrobenzène, le 2-(β-hydroxyéthyl) amino-6-(β-hydroxyéthyl) amino-4-méthoxynitrobenzène, le 2-[(3-méthylamino-5-méthylamino-4-nitro)phénoxy] éthanol, le 3-[(3-méthylamino-5-méthylamino-4-nitro) phénoxy] propane 1,2-diol, le 2-méthylamino-6-(β-méthoxyéthyl) amino-4-méthoxynitrobenzène, le 4-β-méthoxyéthoxy-2-(β-hydroxyéthyl) amino-6-(β-hydroxyéthyl) aminonitrobenzène, le 2-méthylamino-6-méthylamino-4-(β-hydroxypropyl) aminonitrobenzène, le 2-méthylamino-6-méthylamino-4-(β, γ-dihydroxypropyl) aminonitrobenzène, le 2-méthylamino-6-méthylamino-4-[(β-hydroxyéthoxy) éthylamino] nitrobenzène, le 2-(β-hydroxyéthyl) amino-6-(β-hydroxyéthyl) amino-4-(β-aminoéthyl)aminonitrobenzène, le 2-(β-hydroxyéthyl) amino-4-(β-hydroxyéthyl)amino-6-(β-hydroxyéthyl) aminonitrobenzène, le [3-(β-hydroxyéthyl)amino-5-(β-hydroxyéthyl) amino-4-nitrophényl] β-hydroxyéthylthioéther, le 2-(β, γ-dihydroxypropyl) amino-6-(β, γ-dihydroxypropyl)amino-4-méthoxynitrobenzène, le 2-(γ-hydroxypropyl) amino-4-(γ-hydroxypropyl) amino-6-(γ-hydroxypropyl) aminonitrobenzène, le 2-amino-6-(β-hydroxyéthyl) amino-4-méthoxynitrobenzène, ou l'un des sels cosmétiquement acceptables de ces composés.

10. Composition tinctoriale selon la revendication 8 ou 9, caractérisée par le fait qu'elle contient 0,001 à 5% en poids, et de préférence 0,05 à 2% en poids, par rapport au poids total de la composition, d'au moins un composé de formule (I) ou l'un de ses sels cosmétiquement acceptables.

11. Composition tinctoriale selon l'une quelconque des revendications 8 à 10, caractérisée par le fait qu'elle a un pH compris entre 3 et 11,5, et de préférence compris entre 5 et 11,5.

12. Composition tinctoriale selon l'une quelconque des revendications 8 à 11, caractérisée par le fait que le solvant est choisi parmi l'eau, les alcanols inférieurs, les alcools aromatiques, les polyols et leurs éthers ou leurs mélanges.

13. Composition selon l'une quelconque des revendications 8 à 12, caractérisée par le fait qu'elle contient en outre des adjuvants cosmétiques choisis parmi les agents tensio-actifs anioniques, cationiques, non ioniques, amphotères ou leurs mélanges, les épaississants, les agents dispersants, les agents de pénétration, les séquestrants, les agents filmogènes, les tampons, les parfums, les agents alcalinisants ou acidifiants.

14. Composition selon l'une quelconque des revendications 8 à 13, destinée à être utilisée pour la coloration directe des cheveux humains, caractérisée par le fait qu'elle contient en outre d'autres colorants directs choisis parmi les colorants azoïques, les colorants anthraquinoniques, les indophénols, les indoanilines et les dérivés nitrés de la série benzénique autres que ceux de formule (I).

15. Composition selon l'une quelconque des revendications 8 à 13, destinée à être utilisée comme lotion de mise en plis, caractérisée par le fait qu'elle se présente sous forme d'une solution aqueuse, alcoolique ou hydroalcoolique, contenant au moins une résine cosmétique.

16. Composition selon l'une quelconque des revendications 8 à 13, destinée à être utilisée pour la teinture d'oxydation des cheveux, caractérisée par le fait qu'elle contient en outre des précurseurs de colorants par oxydation et éventuellement des coupleurs.

17. Procédé de coloration directe des fibres kératiniques, en particulier des cheveux humains, caractérisé par le fait qu'on applique sur les fibres une composition telle que définie dans l'une quelconque des revendications 8 à 14, on laisse poser pendant 5 à 50 minutes, on rince, on lave éventuellement au shampooing, on rince à nouveau et on sèche.

18. Procédé de coloration des fibres kératiniques et en particulier des cheveux humains, caractérisé par le fait qu'on applique sur les fibres lavées et rincées une composition telle que définie dans la revendication 15, on enroule éventuellement et on sèche.

19. Procédé de coloration des fibres kératiniques et en particulier des cheveux humains, mettant en oeuvre la révélation par un oxydant, caractérisé par le fait que l'on applique sur les fibres une composition définie dans la revendication 16 mélangée à un oxydant, on laisse poser 10 à 50 minutes, on rince, on lave éventuellement, on rince et on sèche.

**Revendications pour l'Etat contractant l'Espagne**

1. Procédé de préparation d'une 2-nitro métaphénylènediamine substituée de formule (I) :

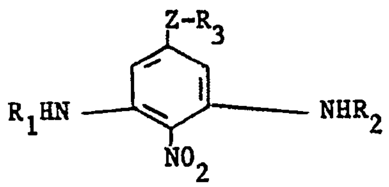

(I)

dans laquelle Z désigne le radical -O-, -S- ou -NH- et $R_1$, $R_2$, et $R_3$, identiques ou différents, désignent un atome d'hydrogène, un radical alkyle, hydroxyalkyle, polyhydroxyalkyle, alcoxyalkyle, hydroxyalcoxyalkyle ou aminoalkyle dont le radical amino peut être mono- ou disubstitué par un radical alkyle ou hydroxyalkyle, les radicaux alkyle et alcoxy comportant 1 à 6 atomes de carbone, caractérisé par le fait qu'il consiste à disubstituer un 2,4,6-trihalogénonitrobenzène de formule (II) :

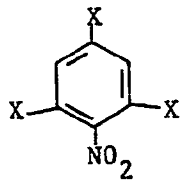

(II)

dans laquelle X représente un atome d'halogène, et en particulier le chlore ou le fluor pour obtenir un 2,6-diamino-4-halogénonitrobenzène de formule (IV) :

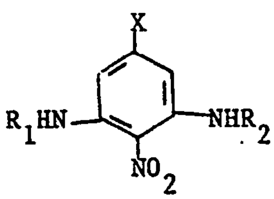

(IV)

dans laquelle $R_1$ et $R_2$ ont les significations indiquées ci-dessus, éventuellement en présence de solvant et à des températures variant entre −10°C et la température de reflux de l'amine $R_1NH_2$ et/ou $R_2NH_2$ utilisée pour la substitution ou de celle du solvant, puis à faire réagir le composé de formule (IV) avec un composé de formule $HZR_3$, en présence ou non de solvant, à une température comprise entre 30 et 150°C.

2. Procédé de préparation du composé de formule (I) selon la revendication 1, caractérisé par le fait que le composé de formule (IV) dans lequel $R_1$ et $R_2$ ont des significations différentes, est préparé en faisant réagir dans un premier temps, l'ammoniac ou une amine de formule $R_1NH_2$, $R_1$ ayant la signification indiquée ci-dessus, sur le 2,4,6-trihalogénonitrobenzène de formule (II) pour conduire au composé de formule (III) :

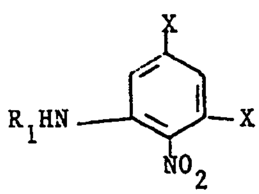

(III)

qui est soumis à l'action d'une amine $R_2NH_2$ ou d'ammoniac pour conduire au composé de formule (IV).

3. Procédé de préparation du composé de formule (I) selon la revendication 1, caractérisé par le fait que le composé (IV) dans lequel le groupement $R_2$ est identique au groupement $R_1$ et différent d'un atome d'hydrogène, est préparé en une seule étape à partir du composé (II) par réaction de ce composé avec une amine de formule $R_1NH_2$.

4. Procédé de préparation du composé de formule (I) selon la revendication 1, dans laquelle $R_1$ et $R_2$ désignent H ou alkyl, $R_3$ = H et Z = 0, caractérisé par le fait que l'on soumet un composé de formule (I) dans laquelle Z = 0 et $R_3$ = $CH_3$ à l'action de l'acide bromhydrique à une température comprise entre 50 et 100°C.

5. Composition tinctoriale pour fibres kératiniques, et en particulier pour cheveux humains, caractérisée par le fait qu'elle contient, dans un milieu solvant, au moins une 2-nitro métaphénylènediamine substituée de formule (I) selon la revendication 1, ou un sel cosmétiquement acceptable de ce composé.

6. Composition tinctoriale selon la revendication 5, caractérisée par le fait qu'elle contient, dans un

milieu solvant, au moins un composé choisi parmi le groupe comprenant le 2,6-diamino-4-méthoxynitrobenzène, le 2,6-diamino-4-hydroxynitrobenzène, le 2-(β-hydroxyéthyl) amino-6-(β-hydroxyéthyl) amino-4-méthoxynitrobenzène, le 2-[(3-méthylamino-5-méthylamino-4-nitro) phénoxy] éthanol, le 3-[(3-méthylamino-5-méthylamino-4-nitro) phénoxy] propane 1,2-diol, le 2-méthylamino-6-(β-méthoxyéthyl) amino-4-méthoxynitrobenzène, le 4-β-méthoxyéthoxy-2-(β-hydroxyéthyl) amino-6-(β-hydroxyéthyl)aminonitrobenzène, le 2-méthylamino-6-méthylamino-4-(β-hydroxypropyl) aminonitrobenzène, le 2-méthylamino-6-méthylamino-4-(β, γ-dihydroxypropyl) aminonitrobenzène, le 2-méthylamino-6-méthylamino-4-[(β-hydroxyéthoxy) éthylamino] nitrobenzène, le 2-(β-hydroxyéthyl)amino-6-(β-hydroxyéthyl) amino-4-(β-aminoéthyl) aminonitrobenzène, le 2-(β-hydroxyéthyl) amino-4-(β-hydroxyéthyl) amino-6-(β-hydroxyéthyl) aminonitrobenzène, le [3-(β-hydroxyéthyl) amino-5-(β-hydroxyéthyl) amino-4-nitrophényl] β-hydroxyéthylthioéther, le 2-(β, γ-dihydroxypropyl) amino-6-(β, γ-dihydroxypropyl) amino-4-méthoxynitrobenzène, le 2-(γ-hydroxypropyl) amino-4-(γ-hydroxypropyl) amino-6-(γ-hydroxypropyl) aminonitrobenzène, le 2-amino-6-(β-hydroxyéthyl)amino-4-méthoxynitrobenzène, ou l'un des sels cosmétiquement acceptables de ces composés.

7. Composition tinctoriale selon la revendication 5 ou 6, caractérisée par le fait qu'elle contient 0,001 à 5% en poids, et de préférence 0,05 à 2% en poids, par rapport au poids total de la composition, d'au moins un composé de formule (I) ou l'un de ses sels cosmétiquement acceptables.

8. Composition tinctoriale selon l'une quelconque des revendications 5 à 7, caractérisée par le fait qu'elle a un pH compris entre 3 et 11,5, et de préférence compris entre 5 et 11,5.

9. Composition tinctoriale selon l'une quelconque des revendications 5 à 8, caractérisée par le fait que le solvant est choisi parmi l'eau, les alcanols inférieurs, les alcools aromatiques, les polyols et leurs éthers ou leurs mélanges.

10. Composition selon l'une quelconque des revendications 5 à 9, caractérisée par le fait qu'elle contient en outre des adjuvants cosmétiques choisis parmi les agents tensio-actifs anioniques, cationiques, non ioniques, amphotères ou leurs mélanges, les épaississants, les agents dispersants, les agents de pénétration, les séquestrants, les agents filmogènes, les tampons, les parfums, les agents alcalinisants ou acidifiants.

11. Composition selon l'une quelconque des revendications 5 à 10, destinée à être utilisée pour la coloration directe des cheveux humains, caractérisée par le fait qu'elle contient en outre d'autres colorants directs choisis parmi les colorants azoïques, les colorants anthraquinoniques, les indophénols, les indoanilines et les dérivés nitrés de la série benzénique autres que ceux de formule (I).

12. Composition selon l'une quelconque des revendications 5 à 10, destinée à être utilisée comme lotion de mise en plis, caractérisée par le fait qu'elle se présente sous forme d'une solution aqueuse, alcoolique ou hydroalcoolique, contenant au moins une résine cosmétique.

13. Composition selon l'une quelconque des revendications 5 à 10, destinée à être utilisée pour la teinture d'oxydation des cheveux, caractérisée par le fait qu'elle contient en outre des précurseurs de colorants par oxydation et éventuellement des coupleurs.

14. Procédé de coloration directe des fibres kératiniques, en particulier des cheveux humains, caractérisé par le fait qu'on applique sur les fibres une composition telle que définie dans l'une quelconque des revendications 5 à 11, on laisse poser pendant 5 à 50 minutes, on rince, on lave éventuellement au shampooing, on rince à nouveau et on sèche.

15. Procédé de coloration des fibres kératiniques et en particulier des cheveux humains, caractérisé par le fait qu'on applique sur les fibres lavées et rincées une composition telle que définie dans la revendication 12, on enroule éventuellement et on sèche.

16. Procédé de coloration des fibres kératiniques et en particulier des cheveux humains, mettant en oeuvre la révélation par un oxydant, caractérisé par le fait que l'on applique sur les fibres une composition définie dans la revendication 13 mélangée à un oxydant, on laisse poser 10 à 50 minutes, on rince, on lave éventuellement, on rince et on sèche.

17. Procédé de préparation d'une composition tinctoriale pour fibres kératiniques, et en particulier pour cheveux humains, caractérisé par le fait qu'il consiste à dissoudre dans un milieu solvant choisi parmi l'eau, les alcanols inférieurs, les alcools aromatiques, les polyols et leurs éthers ou leur mélange, 0,001 à 5% en poids, et de préférence 0,05 à 2% en poids, par rapport au poids total de la composition, d'au moins une 2-nitrométaphénylènediamine substituée de formule (I) selon la revendication 1, ou d'un de ses sels cosmétiquement acceptables, à ajouter ensuite au moins un adjuvant cosmétique choisi parmi les agents tensio-actifs anioniques, cationiques, non-ioniques, amphotères ou leurs mélanges, les épaississants, les agents dispersants, les agents de pénétration, les séquestrants, les agents filmogènes, les tampons et les parfums, et enfin à ajuster le pH de la composition de teinture à une valeur comprise entre 3 et 11,5 et de préférence entre 5 et 11,5 à l'aide d'un agent alcalinisant au acidifiant.

18 . Procédé selon la revendication 16, caractérisé par le fait qu'on utilise à titre de composé de formule (I) un composé choisi parmi le groupe comprenant le 2,6-diamino-4-méthoxynitrobenzène, le 2,6-diamino-4-hydroxynitrobenzène, le 2-(β-hydroxyéthyl) amino-6-(β-hydroxyéthyl) amino-4-méthoxynitrobenzène, le 2-[(3-méthylamino-5-méthylamino-4-nitro) phénoxy] éthanol, le 3-[(3-méthylamino-5-méthylamino-4-nitro) phénoxy] propane 1,2-diol, le 2-méthylamino-6-(β-méthoxyéthyl) amino-4-méthoxynitrobenzène, le 4-β-méthoxyéthoxy-2-(β-hydroxyéthyl) amino-6-(β-hydroxyéthyl) aminonitrobenzène, le 2-méthylamino-6-méthylamino-4-(β-hydroxypropyl) aminonitrobenzène, le 2-méthylamino-6-méthylamino-4-(β, γ -dihydroxypropyl) aminonitrobenzène, le 2-méthylamino-6-méthylamino-4- [(β-hydroxyéthoxy) éthylamino] nitrobenzène, le 2-(β-hydroxyéthyl) amino-6-(β-hydroxyéthyl)amino-4-(β-aminoéthyl) aminonitrobenzène, le 2-(β-hydroxyéthyl)amino-4-(β-hydroxyéthyl) amino-6-(β-hydroxyéthyl) aminonitrobenzène, le [3-(β-hydroxyéthyl) amino-5-(β-hydroxyéthyl) amino-4-nitrophényl] β-hydroxyéthylthioéther, le 2-(β, γ -dihydroxypropyl) amino-6-(β, γ-dihydroxypropyl) amino-4-méthoxynitrobenzène, le 2-(γ-hydroxypropyl) amino-4-(γ-hydroxypropyl) amino-6-(γ-hydroxypropyl)aminonitrobenzène, le 2-amino-6-(β-hydroxyéthyl) amino-4-méthoxynitrobenzène, ou l'un des sels cosmétiquement acceptables de ces composés.

19. Procédé de préparation d'une composition tinctoriale pour cheveux humains selon la revendication 17 ou 18, destinée à être utilisée pour la coloration directe des cheveux, caractérisé par le fait qu'on dissout en outre dans le milieu solvant d'autres colorants directs choisis parmi les colorants azoïques, les colorants anthraquinoniques, les indophénols, les indoanilines et les dérivés nitrés de la série benzénique autre que ceux de formule (I).

20. Procédé de prépration d'une composition tinctoriale pour cheveux humains selon la revendication 17 ou 18, destinée à être utilisée pour la teinture d'oxydation des cheveux, caractérisé par le fait qu'on dissout entre outre dans le milieu solvant des précurseurs de colorants pour oxydation et éventuellement des coupleurs.

## Ansprüche

**Patentansprüche für die benannten Vertragsstaaten : AT, BE, CH, DE, FR, GB, GR, IT, LI, NL, SE**

1. Substituiertes 2-Nitro-m-phenylendiamin der Formel (I)

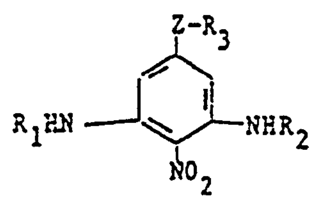

(I)

worin Z den Rest -O-, -S- oder -NH- und $R_1$, $R_2$ und R3, gleich oder verschieden, ein Wasserstoffatom, einen Alkyl-, Hydroxyalkyl-, Polyhydroxyalkyl-, Alkoxyalkyl-, Hydroxyalkoxyalkyl- oder Aminoalkylrest darstellen, dessen Aminorest durch einen Alkyl- oder Hydroxyalkylrest mono- oder disubstituiert sein kann, wobei die Alkyl- und Alkoxyreste 1 bis 6 Kohlenstoffatome enthalten, oder die kosmetisch verträglichen Salze dieser Verbindung.

2. Verbindung gemäß Anspruch 1, dadurch **gekennzeichnet**, daß $R_1$, $R_2$ und $R_3$, gleich oder verschieden, ein Wasserstoffatom, einen Methyl-, Ethyl-, n-Propyl-, n-Butyl-, beta-Hydroxyethyl-, beta-Hydroxypropyl-, gamma-Hydroxypropyl-, beta, gamma-Dihydroxypropyl-, Methoxyethyl-, Ethoxyethyl-, beta-Hydroxyethoxyethyl-, beta-Aminoethyl-, beta-Hydroxyethylaminoethyl- oder beta-Diethylaminoethylrest darstellen.

3. Verbindung gemäß den Ansprüchen 1 oder 2, dadurch **gekennzeichnet**, daß sie aus der Gruppe ausgewählt ist, umfassend 2,6-Diamino-4-methoxynitrobenzol, 2,6-Diamino-4-hydroxynitrobenzol, 2-(beta-Hydroxyethyl) amino-6-(beta-hydroxyethyl) amino-4-methoxynitrobenzol, 2-((3-Methylamino-5-methylamino-4-nitro) phenoxy) ethanol, 3-((3-Methylamino-5-methylamino-4-nitro) phenoxy) propan-1,2-diol, 2-Methylamino-6-(beta-methoxyethyl) amino-4-methoxynitrobenzol, 2-Methylamino-6-methylamino-4-(beta-hydroxypropyl) aminonitrobenzol, 2-Methylamino-6-methylamino-4-(beta, gamma-dihydroxypropyl) aminonitrobenzol, 2-Methylamino-6-methylamino-4((beta-hydroxyethoxy) ethylamino) nitrobenzol, 2-(beta-Hydroxyethyl) amino-6-(beta-hydroxyethyl) amino-4(beta-aminoethyl) amino-nitrobenzol, 2-(beta-Hydroxyethyl) amino-4-(beta-hydroxyethyl) amino-6-(beta- hydroxyethyl) aminonitrobenzol, 4-beta-Methoxyethoxy-2-(beta-hydroxyethyl) amino-6-(beta-hydroxyethyl) aminonitrobenzol, (3-(beta-Hydroxyethyl) amino-5-(beta-hydroxyethyl) amino-4-nitrophenyl) beta-hydroxyethylthioether, 2-(beta, gamma-Dihydroxypropyl) amino-6-(beta, gamma-dihydr oxypropyl) amino-4-metho-xynitrobenzol, 2-(gamma-Hydroxypropyl) amino-4-(gamma-hydroxypropyl) amin o-6-(gamma-hydroxy- propyl) aminonitrobenzol und 2-Amino-6-(beta-hydroxyethyl) amino-4-methoxynitrobenzol.

4. Verfahren zur Herstellung eines substituierten 2-Nitro-m-phenylendiamins der Formel (I) gemäß Anspruch 1, dadurch **gekennzeichnet**, daß man ein 2,4,6-Trihalogennitrobenzol der Formel (II) :

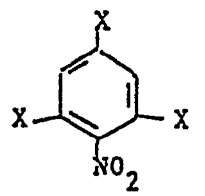

(II)

worin X ein Halogenatom und insbesondere Chlor oder Fluor darstellt, disubstituiert, um ein 2,6-Diamino-4-halogennitrobenzol der Formel (IV) zu erhalten :

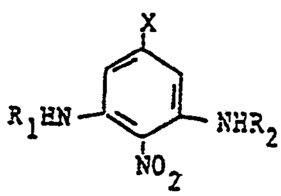

(IV)

worin $R_1$ und $R_2$ die in Anspruch 1 angegebenen Bedeutungen haben, und zwar gegebenenfalls in Anwesenheit von Lösungsmittel und bei Temperaturen zwischen −10°C und der Rückflußtemperatur des für die Substitution verwendeten Amins $R_1NH_2$ und/oder R2NH2 oder derjenigen des Lösungsmittels, worauf man die Verbindung der Formel (IV) mit einer Verbindung der Formel $HZR_3$, in Anwesenheit von Lösungsmittel oder nicht, bei einer Temperatur zwischen 30 und 150°C reagieren läßt.

5. Verfahren zur Herstellung der Verbindung der Formel (I) gemäß Anspruch 4, dadurch **gekennzeichnet**, daß die Verbindung der Formel (IV), in der $R_1$ und $R_2$ verschiedene Bedeutungen haben, hergestellt wird, indem man zuerst Ammoniak oder ein Amin der Formel $R_1NH_2$, wobei $R_1$ die oben angegebene Bedeutung hat, mit 2,4,6-Trihalogennitrobenzol der Formel (II) reagieren läßt, um zur Verbindung der Formel (III) zu führen :

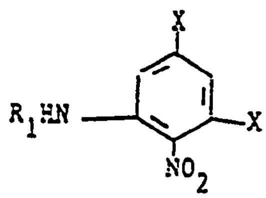

(III)

welche dann der Einwirkung durch ein Amin $R_2NH_2$ oder von Ammoniak unterworfen wird, um zur Verbindung der Formel (IV) zu führen.

6. Verfahren zur Herstellung der Verbindung der Formel (I) gemäß Anspruch 4, dadurch **gekennzeichnet**, daß die Verbindung (IV), worin die Gruppe $R_2$ mit Gruppe $R_1$ identisch und von Wasserstoff verschieden ist, in einer einzigen Stufe hergestellt wird, ausgehend von Verbindung (II) durch Reaktion dieser Verbindung mit einem Amin der Formel $R_1NH_2$.

7. Verfahren zur Herstellung der Verbindung der Formel (I) gemäß Anspruch 1, worin $R_1$ und $R_2$ H oder Alkyl darstellen, $R_3$ = H und Z = 0, dadurch **gekennzeichnet**, daß man eine Verbindung der Formel (I), worin Z = 0 und $R_3$ = $CH_3$, der Einwirkung von Bromwasserstoffsäure bei einer Temperatur zwischen 50 und 100°C unterwirft.

8. Färbezusammensetzung für keratinische Fasern und insbesondere für menschliche Haare, dadurch **gekennzeichnet**, daß sie in einem Lösungsmittelmedium mindestens ein substituiertes 2-Nitro-m-phenylendiamin der Formel (I) gemäß Anspruch 1 oder ein kosmetisch verträgliches Salz dieser Verbindung enthält.

9. Färbezusammensetzung gemäß Anspruch 8, dadurch **gekennzeichnet**, daß sie in einem Lösungsmittelmedium mindestens eine Verbindung, ausgewählt aus der Gruppe, umfassend 2,6-Diamino-4-methoxynitrobenzol, 2,6-Diamino-4-hydroxynitrobenzol, 2-(beta-Hydroxyethyl) amino-6-(beta-hydroxyethyl) amino-4-methoxynitrobenzol, 2-((3-Methylamino-5-methylamino-4-nitro) phenoxy) ethanol, 3-((3-Methylamino-5-methylamino-4-nitro) phenoxy) propan-1 ,2-diol, 2-Methylamino-6-(beta-methoxyethyl) amino-4-methoxynitrob enzol, 2-Methylamino-6-methylamino-4-(beta-hydroxypropyl) aminon itrobenzol, 2-Methylamino-6-methylamino-4-(beta, gamma-dihydroxypropy 1) aminonitrobenzol, 2-Methylamino-6-methylamino-4-((beta-hydroxyethoxy) ethyl amino) nitrobenzol, 2-(beta-Hydroxyethyl) amino-6-(beta-hydroxyethyl) amino-4-(beta-aminoethyl) amino-nitrobenzol, 2-(beta-Hydroxyethyl) amino-4-(beta-hydroxyethyl) amino-6-(beta- hydroxyethyl) aminonitrobenzol, 4-beta-Methoxyethoxy-2-(beta-hydroxyethyl) amino-6-(beta-hydroxyethyl) aminonitrobenzol, (3-(beta-Hydroxyethyl) amino-5-(beta-hydroxyethyl) amino-4-nitrophenyl) beta-hydroxyethylthioether, 2-(beta, gamma-Dihydroxypropyl) amino-6-(beta, gamma-dihydr oxypropyl) amino-4-metho-xynitrobenzol, 2-(gamma-Hydroxypropyl) amino-4-(gamma-hydroxypropyl) amin o-6-(gamma-hydroxy- propyl) aminonitrobenzol, 2-Amino-6-(beta-hydroxyethyl) amino-4-methoxynitrobenzol, oder eines der kosmetisch verträglichen Salze dieser Verbindungen enthält.

10. Färbezusammensetzung gemäß Anspruch 8 oder 9, dadurch **gekennzeichnet**, daß sie 0,001 bis 5 Gewichtsprozent, vorzugsweise 0,05 bis 2 Gewichtsprozent, bezogen auf Gesamtgewicht der Zusammensetzung, mindestens einer Verbindung der Formel (I) oder eines ihrer kosmetisch verträglichen Salze enthält.

11. Färbezusammensetzung gemäß jedem der Ansprüche 8 bis 10, dadurch **gekennzeichnet**, daß sie einen pH zwischen 3 und 11,5, vorzugsweise zwischen 5 und 11,5, aufweist.

12. Färbezusammensetzung gemäß jedem der Ansprüche 8 bis 11, dadurch **gekennzeichnet**, daß das Lösungsmittel unter Wasser, Niedrigalkanolen, aromatischen Alkoholen, Polyolen und deren Ethern oder deren Mischungen ausgewählt ist.

13. Zusammensetzung gemäß jedem der Ansprüche 8 bis 12, dadurch **gekennzeichnet**, daß sie außerdem kosmetische Hilfsmittel enthält, ausgewählt unter anionischen, kationischen, nicht-ionischen, amphoteren oberflächenaktiven Mitteln oder deren Mischungen, Verdickungsmitteln, Dispergiermitteln, Penetrationsmitteln, Maskierungsmitteln, filmbildenden Mitteln, Tampons, Parfümen, alkalisch oder sauer machenden Mitteln.

14. Zusammensetzung gemäß jedem der Ansprüche 8 bis 13 zur Verwendung zur Direktfärbung menschlicher Haare, dadurch **gekennzeichnet**, daß sie zusätzlich andere Direktfärbemittel enthält, ausgewählt unter Azofarbstoffen, Anthrachinonfarbstoffen, Indophenolen, Indoanilinen und anderen nitrierten Derivaten der Benzolreihe als denjenigen der Formel (I).

15. Zusammensetzung gemäß jedem der Ansprüche 8 bis 13 zur Verwendung als Haarlegelotion, dadurch **gekennzeichnet**, daß sie in Form einer wässrigen, alkoholischen oder hydroalkoholischen Lösung, enthaltend mindestens ein Kosmetikharz, vorliegt.

16. Zusammensetzung gemäß jedem der Ansprüche 8 bis 13 zur Verwendung zur Oxidationsfärbung von Haaren, dadurch **gekennzeichnet**, daß sie zusätzlich Oxidationsfarbstoffvorstufen und gegebenenfalls Kupplungsmittel enthält.

17. Verfahren zur Direktfärbung keratinischer Fasern und insbesondere menschlicher Haare, dadurch **gekennzeichnet**, daß man auf die Fasern eine Zusammensetzung gemäß jedem der Ansprüche 8 bis 14 aufbringt, während 5 bis 50 Minuten verweilen läßt, die Fasern spült, gegebenenfalls wäscht und schamponiert, erneut spült und trocknet.

18 Verfahren zur Färbung keratinischer Fasern und insbesondere menschlicher Haare, dadurch **gekennzeichnet**, daß man auf die gewaschenen und gespülten Fasern eine Zusammensetzung gemäß Anspruch 15 aufbringt, die Fasern gegebenenfalls einwickelt und trocknet.

19. Verfahren zur Färbung keratinischer Fasern und insbesondere menschlicher Haare, wobei man die Entwicklung durch ein Oxidationsmittel anwendet, dadurch **gekennzeichnet**, daß man auf die Fasern eine Zusammensetzung gemäß Anspruch 16, vermischt mit einem Oxidationsmittel, aufbringt, 10 bis 50 Minuten verweilen läßt, sie spült, gegebenenfalls wäscht, spült und trocknet.

**Patentansprüche für den benannten Vertragsstaat : ES**

1. Verfahren zur Herstellung eines substituierten 2-Nitro-m-phenylendiamins der Formel (I)

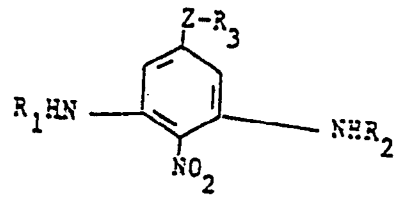

(I)

worin Z den Rest -O-, -S- oder -NH- und $R_1$, $R_2$ und $R_3$, gleich oder verschieden, ein Wasserstoffatom, einen Alkyl-, Hydroxyalkyl-, Polyhydroxyalkyl-, Alkoxyalkyl-, Hydroxyalkoxyalky- oder Aminoalkylrest darstellen, dessen Aminorest durch einen Alkyl- oder Hydroxyalkylrest mono- oder disubstituiert sei kann, wobei die Alkyl- und Alkoxyreste 1 bis 6 Kohlenstoffatome enthalten, dadurch **gekennzeichnet**, daß man 2,4,6-Trihalogennitronenzol der Formel (II)

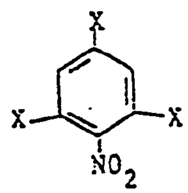

(II)

worin X ein Halogenatom und insbesondere Chlor oder Fluor darstellt, disubstituiert, um 2,6-Diamino-4-halogennitronenzol der Formel (IV) zu erhalten :

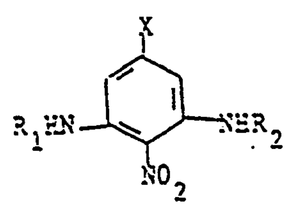

(IV)

worin $R_1$ und $R_2$ die oben angegebenen Bedeutungen haben, und zwar gegebenenfalls in Anwesenheit von Lösungsmittel und bei Temperaturen zwischen −10°C und der Rückflußtemperatur des für die Substitution verwendeten Amins $R_1NH_2$ und/oder $R_2NH_2$ oder derjenigen des Lösungsmittels, worauf man die Verbindung der Formel (IV) mit einer Verbindung der Formel $HZR_3$, in Anwesenheit von Lösungsmittel oder nicht, bei einer Temperatur zwischen 30 und 150°C reagieren läßt.

2. Verfahren zur Herstellung der Verbindung der Formel (I) gemäß Anspruch 1, dadurch **gekennzeichnet**, daß die Verbindung der Formel (IV), worin $R_1$ und $R_2$ verschiedene Bedeutungen haben, hergestellt wird, indem man zuerst Ammoniak oder ein Amin der Formel $R_1NH_2$, wobei $R_1$ die oben angegebene Bedeutung hat, mit 2,4,6-Trihalogennitrobenzol der Formel (II) reagieren läßt, um zur Verbindung der Formel (III) zu führen :

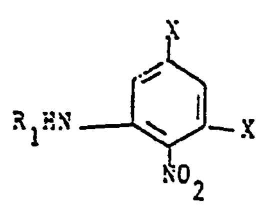

(III)

die dann Einwirkung durch ein Amin $R_2NH_2$ oder von Ammoniak unterworfen wird, um zur Verbindung der Formel (IV) zu führen.

3. Verfahren zur Herstellung der Verbindung der Formel (I) gemäß Anspruch 1, dadurch **gekennzeichnet**, daß die Verbindung (IV), worin die Gruppe $R_2$ mit Gruppe $R_1$ identisch und von wasserstoff verschieden ist, in einer einzigen Stufe hergestellt wird, ausgehend von Verbindung (II) durch Reaktion dieser Verbindung mit einem Amin der Formel $R_1NH_2$.

4. Verfahren zur Herstellung der Verbindung der Formel (I) gemäß Anspruch 1, worin $R_1$ und $R_2$ H oder Alkyl darstellen, $R_3$ = H und Z = 0, dadurch **gekennzeichnet**, daß man eine Verbindung der Formel (I), worin Z = 0 und $R_3$ = $CH_3$, der Einwirkung von Bromwasserstoffsäure bei einer Temperatur zwischen 50 und 100°C unterwirft.

5. Färbezusammensetzung für keratinische Fasern und insbesondere für menschliche Haare, dadurch **gekennzeichnet**, daß sie in einem Lösungsmittelmedium mindestens ein substituiertes 2-Nitro-m-phenylendiamin der Formel (I), erhältlich gemäß Anspruch 1, oder ein kosmetisch verträgliches Salz dieser Verbindung enthält.

6. Färbezusammensetzung gemäß Anspruch 5, dadurch **gekennzeichnet**, daß sie in einem Lösungsmittelmedium mindestens eine Verbindung, ausgewählt aus der Gruppe, umfassend 2,6-Diamino-4-methoxynitrobenzol, 2,6-Diamino-4-hydroxynitronenzol, 2-(beta-Hydroxyethyl) amino-6-(beta-hydroxyethyl) amino-4methoxynitrobenzol, 2-((3-Methylamino-5-methylamino-4-nitro) phenoxy) ethanol, 3-((3-Methylamino-5-methylamino-4-nitro) phenoxy) propan-1, 2-diol, 2-Methylamino-6-(beta-methoxyethyl) ami no-4-methoxynitrobenzol, 2-Methylamino-6-methylamino-4-(beta-hydroxypropyl) aminonitrobenzol, 2-Methylamino-6-methylamino-4-(beta, gamma-dihydroxypropy l) aminonitrobenzol, 2-Methylamino-6-methylamino-4-((beta-hydroxyethoxy) ethylamino) nitrobenzol, 2-(beta-Hydroxyethyl) amino-6-(beta-hydroxyethyl) amino-4-(beta-aminoethyl) amino-nitrobenzol, 2-(beta-Hydroxyethyl) amino-4-(beta-hydroxyethyl) amino-6-(beta- hydroxyethyl) aminonitrobenzol, 4-beta-methoxyethoxy-2-(beta-hydroxyethyl) amino-6-(beta-hydroxyethyl) aminonitronenzol, (3-(beta-Hydroxyethyl) amino-5-(beta-mydroxyethyl) amino-4 -nitrophenyl) beta-hydroxyethylthioether, 2-(beta, gamma-Dihydroxypropyl) amino-6-(beta,gamma-dihydroxypropyl) amino-4-metho-xynitrobenzol, 2-(gamma-Hydroxypropyl) amino-4-(gamma-hydroxypropyl) amin o-6-(gamma-hydroxy- propyl) aminonitrobenzol, 2-Amino-6-(beta-hydroxyethyl) amino-4-methoxynitrobenzol, oder eines der kosmetisch verträglichen Salze dieser Verbindungen enthält.

7. Färbezusammensetzung gemäß Anspruch 5 oder 6, dadurch **gekennzeichnet**, daß sie 0,001 bis 5 Gewichtsprozent, vorzugsweise 0,05 bis 2 Gewichtsprozent, bezogen auf Gesamtgewicht der Zusammensetzung, mindestens einer Verbindung der Formel (I) oder eines ihrer kosmetisch verträglichen Salze enthält.

8. Färbezusammensetzung gemäß jedem der Ansprüche 5 bis 7, dadurch **gekennzeichnet**, daß sie einen pH zwischen 3 und 11,5, vorzugsweise zwischen 5 und 11,5, aufweist.

9. Färbezusammensetzung gemäß jedem der Ansprüche 5 bis 8, dadurch **gekennzeichnet**, daß das Lösungsmittel unter Wasser, Niedrigalkanolen, aromatischen Alkoholen, Polyolen und deren Ethern oder deren Mischungen ausgewählt ist.

10. Zusammensetzung gemäß jedem der Ansprüche 5 bis 9, dadurch **gekennzeichnet**, daß sie außerdem kosmetische Hilfsmittel enthält, ausgewählt unter anionischen, kationischen, nicht-ionischen, amphoteren oberflächenaktiven Mitteln oder deren Mischungen, Verdickungsmitteln, Dispergiermitteln, Penetrationsmitteln, Maskierungsmitteln, filmbildenden Mitteln, Tampons, Parfümen, alkalisch oder sauer machenden Mitteln.

11. Zusammensetzung gemäß jedem der Ansprüche 5 bis 10 zur Verwendung zur Direktfärbung menschlicher Haare, dadurch **gekennzeichnet**, daß sie zusätzlich andere Direktfärbemittel enthält, ausgewählt unter Azofarbstoffen, Anthrachinonfarbstoffen, Indophenolen, Indoanilinen und anderen nitrierten Derivaten der Benzolreihe als denjenigen der Formel (I).

12. Zusammensetzung gemäß jedem der Ansprüche 5 bis 10 zur Verwendung als Haarlegelotion, dadurch **gekennzeichnet**, daß sie in Form einer wässrigen, alkoholischen oder hydroalkoholischen Lösung, enthaltend mindestens ein Kosmetikharz, vorliegt.

13. Zusammensetzung gemäß jedem der Ansprüche 5 bis 10 zur Verwendung zur Oxidationsfärbung von Haaren, dadurch **gekennzeichnet**, daß sie zusätzlich Oxidationsfarbstoffvorstufen und gegebenenfalls Kupplungsmittel enthält.

14. Verfahren zur Direktfärbung keratinischer Fasern und insbesondere menschlicher Haare, dadurch **gekennzeichnet**, daß man auf die Fasern eine Zusammensetzung gemäß jedem der Ansprüche 5 bis 11 aufbringt, während 5 bis 50 Minuten verweilen läßt, die Fasern spült, gegebenenfalls wäscht und schamponiert, erneut spült und trocknet.

15. Verfahren zur Färbung keratinischer Fasern und insbesondere menschlicher Haare, dadurch **gekennzeichnet**, daß man auf die gewaschenen und gespülten Fasern eine Zusammensetzung gemäß

Anspruch 12 aufbringt, die Fasern gegebenenfalls einwickelt und trocknet.

16. Verfahren zur Färbung keratinischer Fasern und insbesondere menschlicher Haare, wobei man die Entwicklung durch ein Oxidationsmittel anwendet, dadurch **gekennzeichnet**, daß man auf die Fasern eine Zusammensetzung gemäß Anspruch 13, vermischt mit einem Oxidationsmittel, aufbringt, 10 bis 50 Minuten verweilen lädt, sie spült, gegebenenfalls wäscht, spült und trocknet.

17. Verfahren zur Herstellung einer Färbezusammensetzung für keratinische Fasern und insbesondere für menschliche Haare, dadurch **gekennzeichnet**, daß man in einem Lösungsmittelmedium, ausgewählt unter Wasser, Niedrigalkanolen, aromatischen Alkoholen, Polyolen und deren Ethern oder deren Mischungen, 0,001 bis 5 Gewichtsprozent, vorzugsweise 0,05 bis 2 Gewichtsprozent, bezogen auf Gesamtgewicht der Zusammensetzung, mindestens eines substituierten 2-Nitro-m-phenylendiamins der Formel (I), erhältlich gemäß Anspruch 1, oder eines seiner kosmetisch verträglichen Salze auflöst, um danach mindestens ein kosmetisches Hilfsmittel, ausgewählt unter anionischen, kationisehen, nicht-ionischen, amphoteren oberflächenaktiven Mitteln oder deren Mischungen, Verdickungsmitteln, Dispergiermitteln, Penetrationsmitteln, Maskierungsmitteln, filmbildenden Mitteln, Tampons und Parfümen, zuzufügen und schließlich den pH der Färbezusammensetzung auf einen Wert zwischen 3 und 11,5 und vorzugsweise zwischen 5 und 11,5 mit Hilfe eines alkalisch oder sauer machenden Mittels einstellt.

18. Verfahren gemäß Anspruch 17, dadurch **gekennzeichnet**, daß man als Verbindung der Formel (I) eine Verbindung, ausgewählt aus der Gruppe, umfassend 2,6-Diamino-4-methoxynitrobenzol, 2,6-Diamino-4-hydroxynitrobenzol, 2-(beta-Hydroxyethyl) amino-6(beta-hydroxyethyl) amino-4-methoxynitrobenzol, 2-((3-Methylamino-5-methylamino-4-nitro) phenoxy) ethanol, 3-((3-Methylamino-5-methylamino-4-nitro) phenoxy) propan-1,2-diol, 2-Methylamino-6-(beta-methoxyethyl) amino-4-methoxynitrobenzol, 2-Methylamino-6-methylamino-4-(beta-hydroxypropyl) aminonitrobenzol, 2-Methylamino-6-methylamino-4-(beta, gamma-dihydroxypropyl) aminonitrobenzol, 2-Methylamino-6-methylamino-4-((beta-hydroxyethoxy) ethyl amino) nitrobenzol, 2(beta-Hydroxyethyl) amino-6-(beta-hydroxyethyl) amino-4-(betaaminoethyl) aminonitrobenzol, 2-(beta-Hydroxyethyl) amino-4-(beta-hydroxyethyl) amino-6-(beta-hydroxyethyl) aminonitrobenzol, 4-beta-Methoxyethoxy-2-(beta-hydroxyethyl) amino-6-(beta-hydroxyethyl) aminonitrobenzol, (3(beta-Hydroxyethyl) amino-5-(beta-hydroxyethyl) amino-4-nitrophenyl) beta-hydroxyethylthioether, 2--(beta, gamma-Dihydroxypropyl) amino-6-(beta, gamma-dihydroxypropyl) amino-4-methoxynitrobenzol, 2-(gamma-Hydroxypropyl) amino-4-(gamma-hydroxypropyl) amino 6-(gamma-hydroxy-propyl) aminonitrobenzol, 2-Amino-6-(beta-hydroxyethyl) amino-4-methoxynitrobenzol, oder eines der kosmetisch verträglichen Salze dieser Verbindungen verwendet.

19. Verfahren zur Herstellung einer Färbezusammensetzung für menschliche Haare gemäß Anspruch 17 oder 18 zur Verwendung zur Direktfärbung von Haaren, dadurch **gekennzeichnet**, daß man in dem Lösungsmittelmedium andere Direktfarbstoffe zusätzlich auflöst, ausgewählt unter Azofarbstoffen, Antrachinonfarbstoffen, Indophenolen, Indoanilinen und anderen nitrierten Derivaten der Benzolreihe als denjenigen der Formel (I).

20. Verfahren zur Herstellung einer Färbezusammensetzung für menschliche Haare gemäß Anspruch 17 oder 18 zur Verwendung für die Oxidationsfärbung von Haaren, dadurch **gekennzeichnet**, daß man zusätzlich in dem Lösungsmittelmedium Oxidationsfarbstoffvorstufen und gegebenenfalls Kupplungsmittel auflöst.

**Claims**

**Claims for the contracting states : AT, BE, CH, DE, FR, GB, GR, IT, LI, NL, SE**

1. Substituted 2-nitro-meta-phenylenediamine of formula :

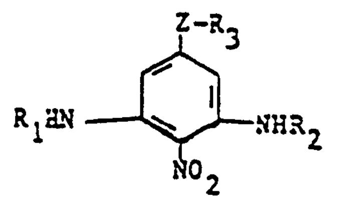

(I)

in which Z denotes an -O-, -S- or -NH- radical and $R_1$, $R_2$ and $R_3$, which may be identical or different, denote a hydrogen atom or an alkyl, hydroxyalkyl, polyhydroxy alkyl, alkoxyalkyl, hydroxyalkoxyalkyl or aminoalkyl radical in which the amino radical may be mono- or disubstituted with an alkyl or hydroxyalkyl radical, the

alkyl and the alkoxy radicals containing 1 to 6 carbon atoms, or the cosmetically acceptable salts of this compound.

2. Compound according to Claim 1, characterized in that $R_1$, $R_2$ and $R_3$, which may be identical or different, denote a hydrogen atom or a methyl, ethyl, n-propyl, n-butyl, β-hydroxyethyl, β-hydroxypropyl, γ-hydroxypropyl, β,γ-dihydroxypropyl, methoxyethyl, ethoxyethyl, β-hydroxyethoxyethyl, β-aminoethyl, β-hydroxyethylamino ethyl or β-diethylaminoethyl radical.

3. Compound according to Claim 1 or 2, characterized in that it is selected from the group comprising 2,6 diamino-4-methoxynitrobenzene, 2,6-diamino-4-hydroxy nitrobenzene, 2-(β-hydroxyethyl) amino-6-(β-hydroxyethyl) amino-4-methoxynitrobenzene, 2-[(3-methylamino-5-methylamino-4-nitro) phenoxy] ethanol, 3[(3-methylamino-5-methy1amino-4-nitro) phenoxy] propane-1,2-diol, 2-methyl- amino-6-(β-methoxyethyl) amino-4-methoxynitrobenzene, 4-(β-methoxyethoxy) -2-(β-hydroxyethyl) amino-6-(β-hydroxyethyl) aminonitrobenzene, 2-methylamino-6-methylamino-4-(β-hydroxypropyl) aminonitrobenzene, 2-methylamino-6methylamino-4-(β, γ-dihydroxypropyl) aminonitrobenzene, 2-methylamino-6-methylamino-4-[(β-hydroxyethoxy) ethylamino) nitrobenzene, 2-(β-hydroxyethyl) amino-6-(β-hydroxyethyl) amino-4-(β-aminoethyl) aminonitrobenzene, 2-(β-hydroxyethyl) amino-4-(β-hydroxyethyl) amino-6-(β-hydroxyethyl) aminonitrobenzene, 3-(β-hydroxyethyl) amino-5-(β-hydroxyethyl) amino-4-nitrophenyl β-hydroxyethyl thioether, 2-(β, γ-dihydroxypropyl) amino-6-(β, γ-dihydroxypropyl) amino-4-methoxynitrobenzene, 2-(γ-hydroxypropyl)amino-4-(γ-hydroxypropyl) amino-6-(γ-hydroxypropyl) aminonitrobenzene and 2-amino-6-(β-hydroxyethyl) amino-4-methoxynitrobenzene.

4. Process for preparing a substituted 2-nitro-metaphenylenediamine of formula (I) according to Claim 1, characterized in that it consists in disubstituting a 2,4,6-trihalonitrobenzene of formula (II) :

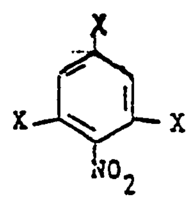

(II)

in which X represents a halogen atom, and especially chlorine or fluorine, to obtain a 2,6-diamino-4-halonitrobenzene of formula (IV) :

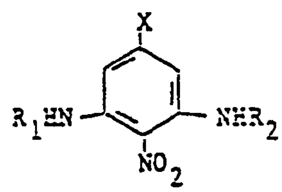

(IV)

in which $R_1$ and $R_2$ have the meanings stated in Claim 1, optionally in the presence of a solvent at temperatures varying between −10°C and the refluxing temperature of the amine $R_1NH_2$ and/or $R_2NH_2$ used for the substitution or of that of the solvent, and then in reacting the compound of formula (IV) with a compound of formula $HZR_3$, in the presence or absence of a solvent, at a temperature of between 30 and 150°C.

5. Process for preparing the compound of formula (I) according to Claim 4, characterized in that the compound of formula (IV) in which $R_1$ and $R_2$ have different meanings is prepared by reacting, in a first stage, ammonia or an amine of formula $R_1NH_2$, $R_1$ having the meaning defined above, with the 2,4,6-trihalonitrobenzene of formula (II) to lead to the compound of formula (III) :

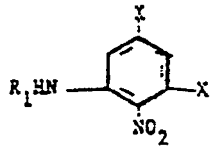

(III)

which is subjected to the action of an amine $R_2NH_2$ or of ammonia to lead to the compound of formula (IV).

6. Process for preparing the compound of formula (I) according to Claim 4, characterized in that the compound (IV) in which the group $R_2$ is identical to the group R, and other than a hydrogen atom is prepared in a single step from the compound (II) by reaction of this compound with an amine of formula $R_1NH_2$.

7. Process for preparing the compound of formula (I) according to Claim 1 in which $R_1$ and $R_2$ denote H or alkyl, $R_3$ = H and Z = O, characterized in that a compound of formula (I) in which Z = O and $R_3$ = $CH_3$ is subjected to the action of hydrobromic acid at a temperature of between 50 and 100°C.

8. Dyeing composition for keratinous fibres and especially for human hair, characterized in that it contains, in a solvent medium, at least one substituted 2-nitro-meta-phenylenediamine of formula (I) according to Claim 1, or a cosmetically acceptable salt of this compound.

9. Dyeing composition according to Claim 8, characterized in that it contains, in a solvent medium, at least one compound selected from the group comprising 2,6-diamino-4-methoxynitrobenzene, 2,6-diamino-4-hydroxynitrobenzene, 2-(β-hydroxyethyl) amino-6-(β-hydroxyethyl)amino-4-methoxynitrobenzene, 2-[(3-methylamino-5-methylamino-4-nitro) phenoxy] ethanol, 3-[(3-methylamino-5-methylamino-4-nitro) phenoxy] propane-1,2-diol, 2-methyl- amino-6-(β-methoxyethyl) amino-4-methoxynitrobenzere, 4-(β-methoxyethoxy)-2-(β-hydroxyethyl) amino-6-(β-hydroxyethyl) aminonitrobenzene, 2-methylamino-6-methylamino-4-(β-hydroxypropyl) aminonitrobenzene, 2-methylamino-6-methylamino-4-(β, γ-dihydroxypropyl) aminonitrobenzene, 2-methylamino-6-methylamino-4-[(γ-hydroxyethoxy) ethyl amino] nitrobenzene, 2-(β-hydroxyethyl) amino-6-(β-hydr-oxyethyl) amino-4-(β-aminoethyl) aminonitrobenzene, 2-(βhydroxyethyl) amino-4-(β-hydroxyethyl) amino-6-(β-hydroxy ethyl) aminonitrobenzene, 3(β-hydroxyethyl) amino-5-(β-hydroxyethyl) amino-4-nitrophenyl β-hydroxyethyl thio-ether, 2-(β, γ- dihydroxypropyl) amino-6-(β, γ-dihydroxypropyl) amino-4-methoxypitrobenzene, 2-(γ-hydroxypropyl)amino-4(γ-hydroxypropyl) amino-6-(γ-hydroxypropyl)-aminonitrobenzene and 2-amino-6-(β-hydroxyethyl) amino-4-methoxynitrobenzene, or one of the cosmetically acceptable salts of these compounds.

10. Dyeing composition according to Claim 8 or 9, characterized in that it contains 0.001 to 5% by weight, and preferably 0.05 to 2 % by weight, relative to the total weight of the composition, of at least one compound of formula (I) or one of its cosmetically acceptable salts.

11. Dyeing composition according to any one of Claims 8 to 10, characterized in that it has a pH of between 3 and 11.5, and preferably between 5 and 11.5.

12. Dyeing composition according to any one of Claims 8 to 11, characterized in that the solvent is selected from water, lower alkanols, aromatic alcohols, polyols and their ethers or mixtures thereof.

13. Composition according to any one of Claims 8 to 12, characterized in that it contains, in addition, cosmetic adjuvants selected from anionic, cationic, nonionic or amphoteric surfactants or mixtures thereof, thickeners, dispersing agents, penetrating agents, sequestering agents, film-forming agents, buffers, fragrances and alkalinizing or acidifying agents.

14. Composition according to any one of Claims 8 to 13, intended for use for the direct dyeing of human hair, characterized in that it contains, in addition, other direct dyes selected from azo dyes, anthraquinone dyes, indophenols, indoanilines and nitro deriratives of the benzene series other than those of formula (I).

15. Composition according to any one of Claims 8 to 13, intended for use as a hair setting lotion, characterized in that it is presented in the form of an aqueous, alcoholic or aqueous-alcoholic solution containing at least one cosmetic resin.

16. Composition according to any one of Claims 8 to 13, intended for use for the oxidation dyeing of hair, characterized in that it contains, in addition, oxidation dye precursors and, optionally, couplers.

17. Process for the direct dyeing of keratinous fibres, especially human hair, characterized in that a composition as defined in any one of Claims 8 to 14 is applied on the fibres and is left in place for 5 to 50 minutes, and the hair is rinsed, optionally washed with shampoo, rinsed again and dried.

18. Process for dyeing keratinous fibres, and especially human hair, characterized in that a composition as defined in Claim 15 is applied on the washed and rinsed fibres, and the hair is optionally wound on rollers and is dried.

19. Process for dyeing keratinous fibres, and especially human hair, employing development with an oxidizing agent, characterized in that a composition as defined in Claim 16, mixed with an oxidizing agent, is applied on the fibres and is left in place for 10 to 50 minutes, and the hair is rinsed, optionally washed, rinsed and dried.

**Claims for the contracting state : ES**

1. Process for preparing a substituted 2-nitro-meta-phenylenediamine of formula 1 :

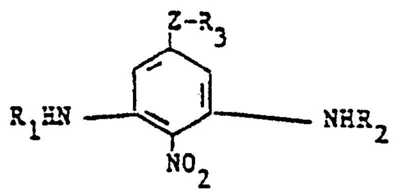

(I)

in which Z denotes an -O-, -S- or -NH- radical and $R_1$, $R_2$ and $R_3$, which may be identical or different, denote a hydrogen atom or an alkyl, hydroxyalkyl, polyhydroxy alkyl, alkoxyalkyl, hydroxyalkoxyalkyl or aminoalkyl radical in which the amino radical may be mono- or disubstituted with an alkyl or hydroxyalkyl radical, the alkyl and the alkoxy radicals containing 1 to 6 carbon atoms, characterized in that it consists in disubstituting a 2,4,6-trihalonitrobenzene of formula (II) :

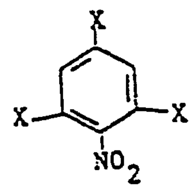

(II)

in which X represents a halogen atom, and especially chlorine or fluorine, to obtain a 2,6-diamino-4-halonitrobenzene of formula (IV) :

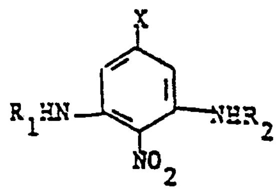

(IV)

in which $R_1$ and $R_2$ have the meanings stated above, optionally in the presence of a solvent at temperatures varying between −10°C and the refluxing temperature of the amine $R_1NH_2$ and/or $R_2NH_2$ used for the substitution or of that of the solvent, and then in reacting the compound of formula (IV) with a compound of formula $HZR_3$, in the presence or absence of a solvent, at a temperature of between 30 and 150°C.

2. Process for preparing the compound of formula (I), according to Claim 1, characterized in that the compound of formula (IV) in which $R_1$ and $R_2$ have different meanings is prepared by reacting, in a first stage, ammonia or an amine of formula $R_1NH_2$, $R_1$ having the meaning stated above, with the 2,4,6 -trihalonitrobenzene of formula (II) to lead to the compound of formula (III) :

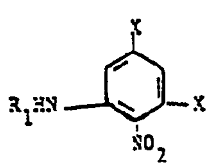

(III)

which is subjected to the action of an amine $R_2NH_2$ or of ammonia to lead to the compound of formula (IV).

3. Process for preparing the compound of formula (I) according to Claim 1, characterized in that the compound (IV) in which the group $R_2$ is identical to the group $R_1$ and other than a hydrogen atom is prepared in a single step from the compound (II) by reaction of this compound with an amine of formula $R_1NH_2$.

4. Process for preparing the compound of formula (I) according to Claim 1 in which $R_1$ and $R_2$ denote H or alkyl, $R_3$ = H and Z = 0, characterized in that a compound of formula (I) in which Z = 0 and $R_3$ = $CH_3$ is subjected to the action of hydrobromic acid at a temperature of between 50 and 100°C.

5. Dyeing composition for keratinous fibres and especially for human hair, characterized in that it contains, in a solvent medium, at least one substituted 2-nitro-meta-phenylenediamine of formula (I) according to Claim 1, or a cosmetically acceptable salt of this compound.

6. Dyeing composition according to Claim 5, characterized in that it contains, in a solvent medium, at least one compound selected from the group comprising 2,6 -diamino-4-methoxynitrobenzene, 2, 6 -diamino-4 -hydroxy nitrobenzene, 2-(β-hydroxyethyl) amino-6-(β-hydroxryethyl)amino-4-methoxynitrobenzene,

2-[(3-methylamino-5-methylamino-4-nitro) phenoxy] ethanol, 3-[(3-methylamino-5-methylamino-4-nitro) phenoxy] propane-1,2-diol, 2-methyl-amino-6-(β-methoxyethyl) amino-4-methoxynitobenzene, 4-(ß-methoxyethoxy)-2-(β-hydroxyethyl) amino-6-(β-hydroxy-ethyl) aminonitrobenzene, 2-methylamino-6-methylamino-4-(β-hydroxyproply) aminonitrobenzene, 2-methylmaino-6-methylamino-4-(β, γ-dihydroxypropyl) aminonitrobenzene, 2-methylamino-6-methylamino-4-[(β-hydroxyethoxy) ethyl-amino) nitrobenzene, 2-(β-hydroxyethyl) amino-6-(β-hydroxyethyl) amino-4-(β-aminoethyl) aminonitrobenzene, 2-(β-hydroxyethyl) amino-4-(β-hydroxyethyl) amino-6-(β-hydroxyethyl) aminonitrobenzene, 3-(β-hydroxyethyl) amino-5-(β-hydroxyethy1) amino-4-nitrophenyl β-hydroxyethyl thioether, 2-(β, γ-dihydroxypropyl) amino-6-(β, γ-dihydroxy-propyl) amino-4-methoxynitrobenzene, 2-(γ-hydroxypropyl)-amino-4-(γ-hydroxypropyl) amino-6-(γ-hydroxypropyl) aminonitrobenzene and 2-amino-6-(β-hydroxyethyl) amino-4-methoxynitrobenzene, or one of the cosmetically acceptable salts of these compounds.

7. Dyeing composition according to Claim 5 or 6, characterized in that it contains 0.001 to 5% by weight, and preferably 0.05 to 2% by weight, relative to the total weight of the composition, of at least one compound of formula (I) or one of its cosmetically acceptable salts.

8. Dyeing composition according to any one of Claims 5 to 7, characterized in that it has a pH of between 3 and 11.5, and preferably between 5 and 11.5.

9. Dyeing composition according to any one of Claims 5 to 8, characterized in that the solvent is selected from water, lower alkanols, aromatic alcohols, polyols and their ethers or mixtures thereof.

10. Composition according to any one of Claims 5 to 9, characterized in that it contains, in addition, cosmetic adjuvants selected from anionic, cationic, nonionic or amphoteric surfactants or mixtures thereof, thickeners, dispersing agents, penetrating agents, sequestering agents, film-forming agents, buffers, fragrances and alkalinizing or acidifying agents.

11. Composition according to any one of Claims 5 to 10, intended for use for the direct dyeing of human hair, characterized in that it contains, in addition, other direct dyes selected from azo dyes, anthraquinone dyes, indophenols, indoanilines and nitro derivatives of the benzene series other than those of formula (I).

12. Composition according to any one of Claims 5 to 10, intended for use as a hair setting lotion, characterized in that it is presented in the form of an aqueous, alcoholic or aqueous-alcoholic solution containing at least one cosmetic resin.

13. Composition according to any one of Claims 5 to 10, intended for use for the oxidation dyeing of hair, characterized in that it contains, in addition, oxidation dye precursors and, optionally, couplers.

14. Process for the direct dyeing of keratinous fibres, especially human hair, characterized in that a composition as defined in any one of Claims 5 to 11 is applied on the fibres and is left in place for 5 to 50 minutes, and the hair is rinsed, optionally washed with shampoo, rinsed again and dried.

15. Process for dyeing keratinous fibres, and especially human hair, characterized in that a composition as defined in Claim 12 is applied on the washed and rinsed fibres, and the hair is optionally wound on rollers and is dried.

16. Process for dyeing keratinous fibres, and especially human hair, employing development with an oxidizing agent, characterized in that a composition as defined in Claim 13, mixed with an oxidizing agent, is applied on the fibres and is left in place for 10 to 50 minutes, and the hair is rinsed, optionally washed, rinsed and dried.

17. Process for preparing a dyeing composition for keratinous fibres, and especially for human hair, characterized in that it consists in dissolving, in a solvent medium selected from water, lower alkanols, aromatic alcohols, polyols and their ethers or a mixture thereof, 0.001 to 5% by weight, and preferably 0.05 to 2 % by weight, relative to the total weight of the composition, of at least one substituted 2-nitro-metaphenylenediamine of formula (I) according to Claim 1, or of one of its cosmetically acceptable salts, and then adding at least one cosmetic adjuvant selected from anionic, cationic, nonionic or amphoteric surfactants or mixtures thereof, thickeners, dispersing agents, penetrating agents, sequestering agents, film-forming agents, buffers and fragrances, and finally in adjusting the pH of the dyeing composition to a value between 3 and 11.5, and preferably between 5 and 11.5, by means of an alkalinizing or acidifying agent.

18. Process according to Claim 16, characterized in that a compound selected from the group comprising 2,6-diamino-4-methoxynitrobenzene, 2,6-diamino-4-hydroxynitrobenzene, 2-(β-hydroxyethyl) amino-6-(β-hydroxyethyl)amino-4-methoxynitrobenzene, 2-(3-methylamino-5-methylamino-4-nitro) phenoxy-3-ethanol, 3-(3-methylamino-5-methylamino-4-nitro) phenoxy) propane-1,2-diol, 2-methylamino-6-(β-methoxyethyl) amino-4-methoxynitrobenzene, 4-(β-methoxyethoxy)-2-(β-hydroxyethyl) amino-6-(β-hydroxyethyl) aminonitrobenzene, 2-methylamino-6-methylamino-4-(β-hydroxypropyl) aminobitrobenzene, 2-methylamino-6-methylamino-4-(β, γ-dihydroxypropyl) aminonitrobenzene, 2-methylamino-6-methylamino-4-[(β-hydroxyethoxy) ethylamino] nitrobenzene, 2-(β-hydroxyethyl) amino-6-(β-hydroxyethyl) amino-4-(β-aminoethyl) aminonitrobenzene, 2-(β-hydroxyethyl) amino-4-(β-hydroxyethyl) amino-6-(β-hydroxyethyl)

aminonitrobenzene, 3-(β-hydroxyethyl) amino-5-(β-hydroxyethyl) amino-4-nitrophenyl β-hydroxyethyl thioether, 2-(β, γ-dihydroxypropyl) amino-6-(β, γ-dihydroxypropyl) amino-4-methoxypitrobenzene, 2-(γ-hydroxypropyl)amino-4-(γ-hydroxypropyl) amino-6-(γ-hydroxypropyl) aminonitrobenzene and 2-amino-6-(β-hydroxyethyl) amino-4-methoxypitrobenzene, or one of the cosmetically acceptable salts of these compounds, is used by way of a compound of formula (I).

19. Process for preparing a dyeing composition for human hair according to Claim 17 or 18, intended for use for the direct dyeing of hair, characterized in that other direct dyes selected from azo dyes, anthraquinone dyes, indophenols, indoanilines and nitro derivatives of the benzene series other than those of formula (I) are dissolved, in addition, in the solvent medium.

20. Process for preparing a dyeing composition for human hair according to Claim 17 or 18, intended for use for the oxidation dyeing of hair, characterized in that oxidation dye precursors and, optionally, couplers are dissolved, in addition, in the solvent medium.